# EUROPEAN PATENT SPECIFICATION

(11) **EP 3 279 963 B1**
(45) Date of publication and mention of the grant of the patent: **09.11.2022**
(21) Application number: 16772246.1
(22) Date of filing: 14.03.2016
(51) Int. Cl.: H01L 51/54, C07D 209/86, C07D 487/04, C09K 11/06

(54) **ORGANIC ELECTROLUMINESCENT ELEMENT**
ORGANISCHES ELEKTROLUMINESZENTES ELEMENT
ÉLÉMENT ÉLECTROLUMINESCENT ORGANIQUE

(30) Priority: 30.03.2015 JP 2015070098
(43) Date of publication of application: 07.02.2018
(73) Proprietor: NIPPON STEEL Chemical & Material Co., Ltd., Tokyo 103-0027 (JP)
(72) Inventor: IKENAGA Yuji, Kitakyushu-shi Fukuoka 804-8503 (JP); KAI Takahiro, Kitakyushu-shi Fukuoka 804-8503 (JP); HOTTA Masanori, Kitakyushu-shi Fukuoka 804-8503 (JP); OGAWA Junya, Kitakyushu-shi Fukuoka 804-8503 (JP); SAKAI Mitsuru, Kitakyushu-shi Fukuoka 804-8503 (JP); TADA Masashi, Kitakyushu-shi Fukuoka 804-8503 (JP); UEDA Tokiko, Kitakyushu-shi Fukuoka 804-8503 (JP); NOGUCHI Katsuhide, Kitakyushu-shi Fukuoka 804-8503 (JP)
(74) Representative: Vossius & Partner Patentanwälte Rechtsanwälte mbB
(86) International application number: PCT/JP2016/058051
(87) International publication number: WO 2016/158363

(56) References cited:
- WO-A1-2009/136596
- WO-A1-2010/098246
- WO-A1-2011/070963
- WO-A1-2011/132683
- WO-A1-2012/014841
- WO-A1-2013/146645
- WO-A1-2014/038677
- WO-A1-2014/097813
- JP-A- 2002 003 833
- KR-A- 20140 138 393

## Description

### Technical Field

The present invention relates to an organic electroluminescent device (hereinafter referred to as "organic EL device"), and more specifically, to an organic EL device that can achieve high efficiency and a long lifetime, while being driven at a low voltage, by using a mixture of compounds each having a specific structure.

### Background Art

In general, an organic EL device includes a light-emitting layer and a pair of counter electrodes interposing the light-emitting layer therebetween in its simplest structure. That is, the organic EL device uses the phenomenon that, when an electric field is applied between both the electrodes, electrons are injected from a cathode and holes are injected from an anode, and each electron and each hole recombine in the light-emitting layer to emit light as energy.

In recent years, progress has been made in developing an organic EL device using an organic thin film. In order to enhance luminous efficiency particularly, the optimization of the kind of electrodes has been attempted for the purpose of improving the efficiency of injection of carriers from the electrodes . As a result, there has been developed a device in which a hole-transporting layer formed of an aromatic diamine and a light-emitting layer-cum-electron-transporting layer formed of an 8-hydroxyquinoline aluminum complex (Alq3) are formed between electrodes as thin films, resulting in a significant improvement in luminous efficiency, as compared to related-art devices in which a single crystal of anthracene or the like is used. Thus, the development of the above-mentioned organic EL device has been promoted in order to accomplish its practical application to a high-performance flat panel having features, such as self-luminescence and rapid response.

Investigations have also been made on using a phosphorescent light-emitting material rather than a fluorescent light-emitting material as an attempt to raise the luminous efficiency of a device. Many kinds of devices including the above-mentioned device in which a hole-transporting layer formed of an aromatic diamine and a light-emitting layer formed of Alq3 are formed use fluorescent light emission. However, by using phosphorescent light emission, that is, by using light emission from a triplet excited state, luminous efficiency is expected to be improved by about three times to four times, as compared to the case of using related-art devices in which fluorescent light (singlet) is used. In order to accomplish this purpose, investigations have been made on adopting a coumarin derivative or a benzophenone derivative as a light-emitting layer, but extremely low luminance has only been provided. After that, investigations have been made on using a europium complex as an attempt to use a triplet state, but highly efficient light emission has not been accomplished. Among the investigations involving using phosphorescent light emission, many investigations on a phosphorescent light-emitting dopant centered on an organometallic complex, such as an iridium complex, have been made, as disclosed in Patent Literature 1, and ones capable of highly efficient light emission have been found.

### Citation List

### Patent Literature

[PTL 1] JP 2003-515897 A
[PTL 2] JP 2001-313178 A
[PTL 3] JP 11-162650 A
[PTL 4] JP 11-176578 A
[PTL 5] WO 2008-056746 A1
[PTL 6] WO 2009-136596 A1
[PTL 7] WO 2010-098246 A1
[PTL 8] WO 2011-132684 A1
[PTL 9] JP 2012-028634 A
[PTL 10] WO 2013-146645 A1
[PTL 11] WO 2014-038677 A1

A host material to be used in the light-emitting layer of the organic EL device is, for example, a carbazole-based compound, an oxazole-based compound, or a triazole-based compound introduced in each of Patent Literatures 1, 2 and 11. However, both the efficiency and lifetime of each of the compounds have not been practical.

In addition, in each of Patent Literatures 3 and 4, there is a disclosure of an indolocarbazole compound. However, it is recommended that the compound be used as a hole-transporting material. In each of the literatures, there is no disclosure of the use of the indolocarbazole compound as a mixed host material, and hence there is no teaching of the usefulness of the indolocarbazole compound as a mixed host material.

In addition, in Patent Literature 5, there is a disclosure of the use of an indolocarbazole compound as a host material. However, in the literature, there is no teaching that the compound has usefulness as a mixed host material.

In addition, in each of Patent Literatures 6 and 7, there is a disclosure of the use of an indolocarbazole compound as a mixed host. However, in each of the literatures, there is no teaching that a useful effect is expressed by combining the compound with a specific carbazole compound.

In addition, in each of Patent Literatures 8, 9 and 10 , there is a disclosure of the use of an indolocarbazole compound and a carbazole compound as a mixed host. However, in each of the literatures, there is no teaching of a useful effect of a combination of a specific indolocarbazole compound and a specific carbazole compound.

### Summary of Invention

In order to apply an organic EL device to a display device in a flat panel display or the like, it is necessary to improve the luminous efficiency of the device and also to ensure sufficiently the stability in driving the device. The present invention has an object to provide, in view of the above-mentioned circumstances, a practically useful organic EL device that has high efficiency and high driving stability while being driven at a low voltage.

According to one aspect of the present invention, there is provided an organic electroluminescent device, including one or more light-emitting layers between an anode and a cathode opposite to each other, in which: at least one of the light-emitting layers contains at least two kinds of host materials and at least one kind of light-emitting dopant; and at least one kind out of the host materials includes a host material (H1) selected from compounds each represented by any one of the following general formulae (1) and (2), and at least another one kind out of the host materials includes a host material (H2) selected from compounds each represented by the following general formula (3).

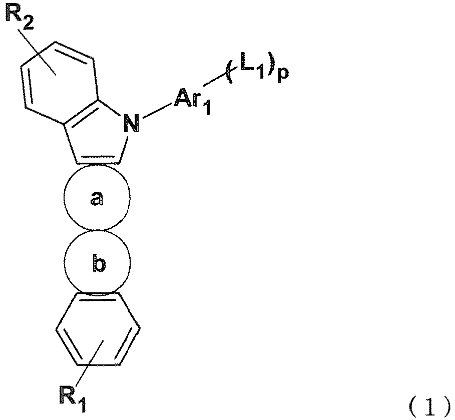

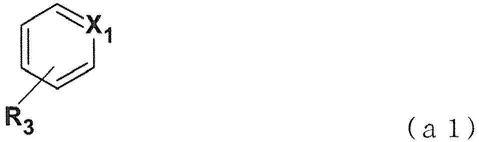

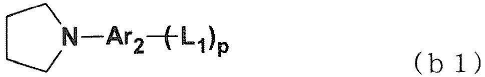

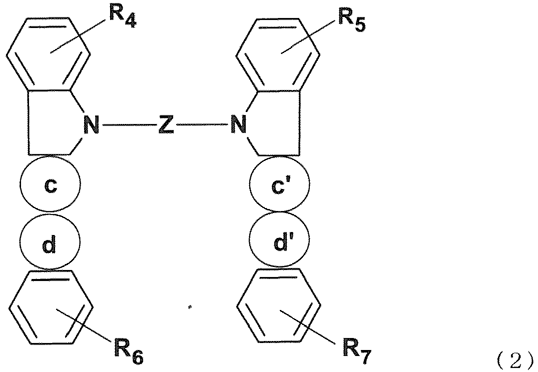

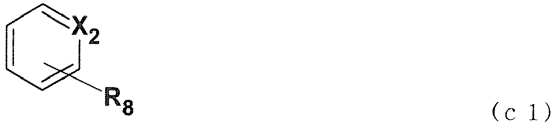

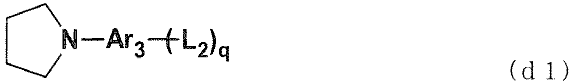

In the general formula (1):
a ring a represents an aromatic ring or a heterocycle represented by the formula (a1) that is fused at arbitrary positions of two adjacent rings, X₁ represents C-R or N, a ring b represents a heterocycle represented by the formula (b1) that is fused at arbitrary positions of two adjacent rings, Ar₁ and Ar₂ each independently represent an aromatic hydrocarbon group having 6 to 24 carbon atoms, or an aromatic heterocyclic group having 3 to 16 carbon atoms, L₁ represents an aromatic hydrocarbon group having 6 to 24 carbon atoms, an aromatic heterocyclic group having 3 to 16 carbon atoms, or a linked aromatic group obtained by linking 2 to 10 aromatic rings of the groups, and the aromatic hydrocarbon group, the aromatic heterocyclic group, or the linked aromatic group in Ar₁, Ar₂, and L₁ may have a substituent; p represents an integer of from 0 to 7, provided that when p represents 2 or more, L₁'s may be identical to or different from each other; and
R and R₁ to R₃ each independently represent hydrogen, an alkyl group having 1 to 20 carbon atoms, an aralkyl group having 7 to 38 carbon atoms, an alkenyl group having 2 to 20 carbon atoms, an alkynyl group having 2 to 20 carbon atoms, a dialkylamino group having 2 to 40 carbon atoms, a diarylamino group having 12 to 44 carbon atoms, a diaralkylamino group having 14 to 76 carbon atoms, an acyl group having 2 to 20 carbon atoms, an acyloxy group having 2 to 20 carbon atoms, an alkoxy group having 1 to 20 carbon atoms, an alkoxycarbonyl group having 2 to 20 carbon atoms, an alkoxycarbonyloxy group having 2 to 20 carbon atoms, an alkylsulfonyl group having 1 to 20 carbon atoms, an aromatic hydrocarbon group having 6 to 24 carbon atoms, or an aromatic heterocyclic group having 3 to 16 carbon atoms, and may each have a substituent.

In the general formula (2):
a ring c and a ring c' each represent an aromatic ring or a heterocycle represented by the formula (c1) that is fused at arbitrary positions of adjacent rings, a ring d and a ring d' each represent a heterocycle represented by the formula (d1) that is fused at arbitrary positions of adjacent rings, and the ring c and the ring c', or the ring d and the ring d' may be identical to or different from each other; X₂ represents C-R' or N, Z represents an aromatic hydrocarbon group having 6 to 24 carbon atoms, an aromatic heterocyclic group having 3 to 16 carbon atoms, or a divalent linked aromatic group obtained by linking 2 to 10 aromatic rings of the groups, Ar₃ represents an aromatic hydrocarbon group having 6 to 24 carbon atoms, or a monocyclic aromatic heterocyclic group having 3 to 6 carbon atoms, L₂ represents an aromatic hydrocarbon group having 6 to 24 carbon atoms, an aromatic heterocyclic group having 3 to 16 carbon atoms, or a linked aromatic group obtained by linking 2 to 10 aromatic rings of the groups, and the aromatic hydrocarbon group, the aromatic heterocyclic group, or the linked aromatic group in Z, Ar₃, and L₂ may have a substituent; q represents an integer of from 0 to 7, provided that when q represents 2 or more, L₂'s may be identical to or different from each other; and
R' and R₄ to R₈ each independently represent hydrogen, an alkyl group having 1 to 20 carbon atoms, an aralkyl group having 7 to 38 carbon atoms, an alkenyl group having 2 to 20 carbon atoms, an alkynyl group having 2 to 20 carbon atoms, a dialkylamino group having 2 to 40 carbon atoms, a diarylamino group having 12 to 44 carbon atoms, a diaralkylamino group having 14 to 76 carbon atoms, an acyl group having 2 to 20 carbon atoms, an acyloxy group having 2 to 20 carbon atoms, an alkoxy group having 1 to 20 carbon atoms, an alkoxycarbonyl group having 2 to 20 carbon atoms, an alkoxycarbonyloxy group having 2 to 20 carbon atoms, an alkylsulfonyl group having 1 to 20 carbon atoms, an aromatic hydrocarbon group having 6 to 24 carbon atoms, or an aromatic heterocyclic group having 3 to 16 carbon atoms, and may each have a substituent.

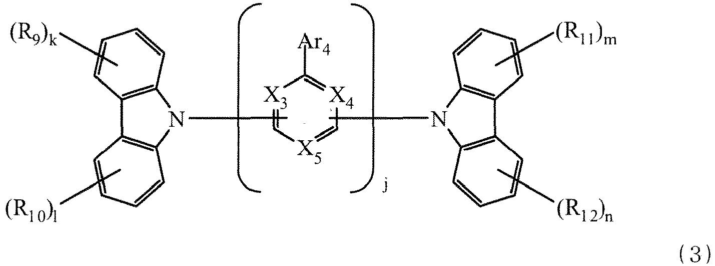

In the formula:
R₉ to R₁₂ each independently represent an aromatic hydrocarbon group having 6 to 18 carbon atoms, or an aromatic heterocyclic group having 3 to 9 carbon atoms, and may each have a substituent;
Ar₄'s each independently represent hydrogen or an aromatic hydrocarbon group having 6 to 24 carbon atoms, and the aromatic hydrocarbon group may have a substituent, j represents an integer of 1 or 3, and at least one Ar₄ does not represent hydrogen;
X₃ to X₅ each independently represent N, C-R", or C-, and R" 's each independently represent hydrogen, an alkyl group having 1 to 20 carbon atoms, an alkoxy group having 1 to 20 carbon atoms, or a diarylamino group having 12 to 44 carbon atoms; and
k, l, m, and n represent integers satisfying 0≤k+l+m+n≤16.

According to another aspect of the present invention, there is provided an organic electroluminescent device, in which a difference in electron affinity (ΔEA) between the two host materials, i.e., the host material (H1) and the host material (H2) is more than 0.1 eV.

In the general formula (1), it is preferred that X₁ represent C-R, and it is more preferred that at least one of Ar₁ or Ar₂ represent a substituted or unsubstituted aromatic heterocyclic group having 3 to 9 carbon atoms.

In the general formula (2), X₂ preferably represents C-R'. In addition, in the general formula (3), j preferably represents an integer of 1.

According to another aspect of the present invention, there is provided a method for preparing an organic electroluminescent device including a light-emitting layer produced by preliminarily mixing the host material (H1) and the host material (H2), and vapor-depositing the mixture from one vapor deposition source. In this case, a mixing ratio of each of the host materials (H1) and (H2) in the light-emitting layer desirably changes by an amount within 5% relative to a preliminary mixing ratio of each of the host materials before the vapor deposition.

In addition, a difference in vaporization temperature between the host material (H1) and the host material (H2) is preferably within 30°C, more preferably within 10°C.

According to another aspect of the present invention, there is provided an organic electroluminescent device, in which the light-emitting dopant includes a phosphorescent light-emitting dopant including an organometallic complex containing at least one metal selected from ruthenium, rhodium, palladium, silver, rhenium, osmium, iridium, platinum, and gold.

Through the use of specific compounds as a mixed host, the organic EL device of the present invention has the lowest excited triplet energy high enough to confine the lowest excited triplet energy of a phosphorescent light-emitting molecule while being driven at a low voltage. Accordingly, energy outflow from the inside of the light-emitting layer of the device is suppressed.

### Brief Description of Drawings

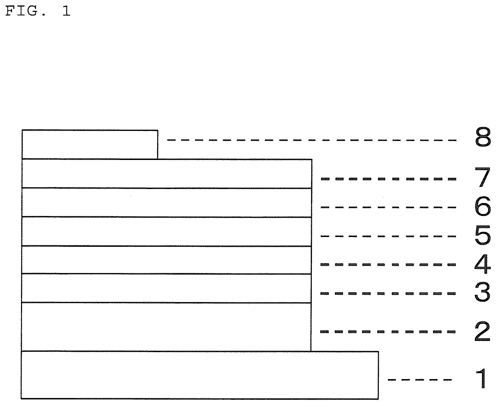

FIG. 1 is a schematic sectional view for illustrating an example of an organic EL device.

### Description of Embodiments

An organic electroluminescent device of the present invention includes one or more light-emitting layers between an anode and a cathode opposite to each other, in which: at least one of the light-emitting layers contains at least two kinds of host materials and at least one kind of light-emitting dopant; and at least one kind out of the host materials includes a host material (H1) selected from compounds each represented by any one of the following general formulae (1) and (2), and at least another one kind out of the host materials includes a host material (H2) selected from compounds each represented by the following general formula (3). The host material (H1) is hereinafter sometimes referred to as "first host material (H1)," and the host material (H2) is hereinafter sometimes referred to as "second host material (H2)." Each of the first host material (H1) and the second host material (H2) may be formed only of one kind of compound, or may be formed of two or more kinds of compounds as long as the compounds satisfy the general formulae (1) and (2), or the general formula (3).

The general formulae (1) and (2) are described below.

A ring a, a ring c, and a ring c' each represent an aromatic ring or a heterocycle represented by the formula (a1) or (c1) that is fused at arbitrary positions of two adjacent rings. Here, in the formula (a1), X₁, which represents C-R or N, preferably represents C-R. In addition, in the formula (c1), X₂, which represents C-R' or N, preferably represents C-R'.

A ring b, a ring d, and a ring d' each represent a heterocycle represented by the formula (b1) or (d1) that is fused at arbitrary positions of two adjacent rings. Here, the ring c and the ring c', or the ring d and the ring d' may be identical to or different from each other.

The aromatic hydrocarbon ring or heterocycle represented by the formula (a1) or (c1) can be fused to two adjacent rings at arbitrary positions but has a position at which the ring or the heterocycle cannot be structurally fused. The aromatic hydrocarbon ring or the heterocycle has six sides but is not fused to the two adjacent rings on two adjacent sides. Similarly, the heterocycle represented by the formula (b1) or (d1) can be fused to two adjacent rings at arbitrary positions but has a position at which the heterocycle cannot be structurally fused. That is, the heterocycle has five sides but is not fused to the two adjacent rings on two adjacent sides. In addition, the heterocycle is not fused to any adjacent ring on a side containing a nitrogen atom. Therefore, the number of kinds of the skeletons of the isomers of the compounds represented by the general formulae (1) and (2) is limited.

Ar₁ to Ar₃ each represent an aromatic hydrocarbon group having 6 to 24 carbon atoms, or an aromatic heterocyclic group having 3 to 16 carbon atoms, and the aromatic hydrocarbon group or the aromatic heterocyclic group may have a substituent.

An aromatic hydrocarbon group having 6 to 24 carbon atoms, or an aromatic heterocyclic group having 3 to 16 carbon atoms is preferred, and an aromatic hydrocarbon group having 6 to 18 carbon atoms, or an aromatic heterocyclic group having 3 to 9 carbon atoms is more preferred. Ar₁ and Ar₂ each represent a (p+1) -valent group, and Ar₃ represents a (q+1)-valent group.

Specific examples of Ar₁ to Ar₃ include groups each produced by removing p+1 or q+1 hydrogen atoms from benzene, pentalene, indene, naphthalene, azulene, heptalene, octalene, indacene, acenaphthylene, phenalene, phenanthrene, anthracene, trindene, fluoranthene, acephenanthrylene, aceanthrylene, triphenylene, pyrene, chrysene, tetraphene, tetracene, pleiadene, picene, perylene, pentaphene, pentacene, tetraphenylene, cholanthrylene, a helicene, hexaphene, rubicene, coronene, trinaphthylene, heptaphene, pyranthrene, furan, thiophene, pyrrole, pyrazole, tellurazole, selenazole, thiazole, isothiazole, oxazole, furazan, thiadiazole, pyridine, pyrazine, pyrimidine, pyridazine, or triazine. Of those, a group produced by removing p+1 or q+1 hydrogen atoms from benzene, naphthalene, anthracene, pyridine, pyrazine, pyrimidine, pyridazine, or triazine is preferred.

In the general formula (1), the formula (b1), and the formula (d1), L₁ and L₂ each represent an aromatic hydrocarbon group having 6 to 24 carbon atoms, an aromatic heterocyclic group having 3 to 16 carbon atoms, or a group obtained by linking 2 to 10 aromatic rings of the groups, and the groups may each have a substituent.

L₁ and L2 each represent preferably an aromatic hydrocarbon group having 6 to 24 carbon atoms, an aromatic heterocyclic group having 3 to 16 carbon atoms, or a linked aromatic group obtained by linking 2 to 10 aromatic rings of the groups, more preferably an aromatic hydrocarbon group having 6 to 18 carbon atoms, an aromatic heterocyclic group having 3 to 16 carbon atoms, or a linked aromatic group obtained by linking 2 to 7 aromatic rings of the groups.

Specific examples of L₁ and L₂ include groups each produced by removing one hydrogen atom from benzene, pentalene, indene, naphthalene, azulene, heptalene, octalene, indacene, acenaphthylene, phenalene, phenanthrene, anthracene, trindene, fluoranthene, acephenanthrylene, aceanthrylene, triphenylene, pyrene, chrysene, tetraphene, tetracene, pleiadene, picene, perylene, pentaphene, pentacene, tetraphenylene, cholanthrylene, a helicene, hexaphene, rubicene, coronene, trinaphthylene, heptaphene, pyranthrene, furan, benzofuran, isobenzofuran, xanthene, oxanthrene, dibenzofuran, peri-xanthenoxanthene, thiophene, thioxanthene, thianthrene, phenoxathiin, thionaphthene, isothianaphthene, thiophthene, thiophanthrene, dibenzothiophene, pyrrole, pyrazole, tellurazole, selenazole, thiazole, isothiazole, oxazole, furazan, pyridine, pyrazine, pyrimidine, pyridazine, triazine, indolizine, indole, isoindole, indazole, purine, quinolizine, isoquinoline, carbazole, imidazole, naphthyridine, phthalazine, quinazoline, benzodiazepine, quinoxaline, cinnoline, quinoline, pteridine, phenanthridine, acridine, perimidine, phenanthroline, phenazine, carboline, phenotellurazine, phenoselenazine, phenothiazine, phenoxazine, anthyridine, benzothiazole, benzimidazole, benzoxazole, benzisoxazole, or benzisothiazole, or an aromatic compound in which a plurality of aromatic rings of these aromatic compounds are linked.

Here, examples of the linking mode of the linked aromatic group represented by any one of L₁ and L₂ in which a plurality of aromatic rings of a plurality of aromatic compounds are linked include the following modes.

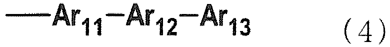

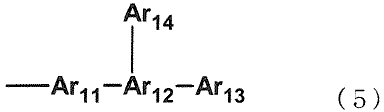

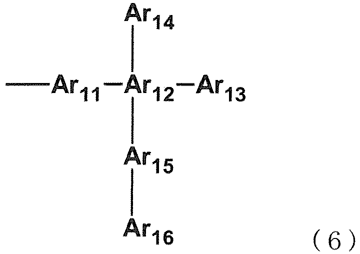

In the formulae (4) to (6), Ar₁₁ to Ar₁₆ each represent a substituted or unsubstituted aromatic ring. The aromatic ring means a ring of an aromatic hydrocarbon compound or an aromatic heterocyclic compound, and can be a group that is monovalent or more. The phrase "aromatic rings are linked" means that the aromatic rings are bonded through a direct bond to be linked. When the aromatic ring is a substituted aromatic ring, a substituent thereof is not an aromatic ring.

Specific examples of the linked aromatic group include groups each produced by removing a hydrogen atom from biphenyl, terphenyl, quaterphenyl, bipyridine, bipyrimidine, bitriazine, terpyridine, phenylterphenyl, binaphthalene, phenylpyridine, diphenylpyridine, phenylpyrimidine, diphenylpyrimidine, phenyltriazine, diphenyltriazine, phenylnaphthalene, diphenylnaphthalene, carbazolylbenzene, biscarbazolylbenzene, biscarbazolyltriazine, dibenzofuranylbenzene, bisdibenzofuranylbenzene, dibenzothiophenylbenzene, or bisdibenzothiophenylbenzene.

In the general formula (2), Z represents an aromatic hydrocarbon group having 6 to 24 carbon atoms, an aromatic heterocyclic group having 3 to 16 carbon atoms, or a divalent linked aromatic group obtained by linking 2 to 10 aromatic rings of the groups. However, a group to be directly linked to N is an aromatic hydrocarbon group having 6 to 24 carbon atoms, or an aromatic heterocyclic group having 3 to 16 carbon atoms. An aromatic hydrocarbon group having 6 to 18 carbon atoms, an aromatic heterocyclic group having 3 to 16 carbon atoms, or a divalent linked aromatic group obtained by linking 2 to 7 aromatic rings of the groups is preferred, and a monocyclic aromatic heterocyclic group is preferably a six-membered ring. The respective aromatic rings may each independently have a substituent.

Specific examples of Z include divalent groups each produced by removing two hydrogen atoms from an aromatic compound listed in the specific examples of L₁ and L₂, or from an aromatic compound in which a plurality of compounds of such kind are linked. However, the group to be linked to N is as described above.

Here, when Z represents a linked aromatic group, examples of its linking mode include the following modes.

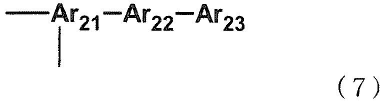

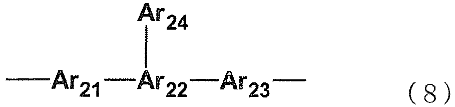

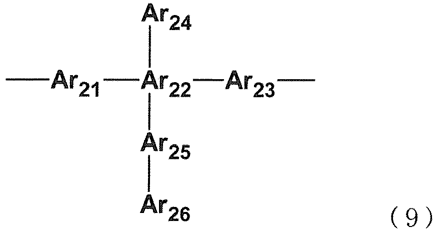

Here, Ar₂₁ to Ar₂₆ each represent a substituted or unsubstituted aromatic ring. The aromatic ring is an aromatic ring forming an aromatic hydrocarbon group or an aromatic heterocyclic group. In addition, instead of a group having a linking hand represented in any one of the formulae, another group can serve as a group having a linking hand as follows: in the formula (7), Ar₂₂; in the formula (8), Ar₂₂ or Ar₂₄; and in the formula (9), Ar₂₄, Ar₂₅, or Ar₂₆. Any such linking hand is bonded to N, and hence a group having the linking hand is an aromatic hydrocarbon group having 6 to 24 carbon atoms, or an aromatic heterocyclic group having 3 to 16 carbon atoms.

p and q each represent an integer of from 0 to 7, preferably from 0 to 5, more preferably from 0 to 3.

In the case where Ar₁ to Ar₃, Z, and L₁ and L₂ each represent an aromatic hydrocarbon group, an aromatic heterocyclic group, or a linked aromatic group, the groups may each have a substituent. In this case, the substituent is an alkyl group having 1 to 20 carbon atoms, an aralkyl group having 7 to 38 carbon atoms, an alkenyl group having 2 to 20 carbon atoms, an alkynyl group having 2 to 20 carbon atoms, a dialkylamino group having 2 to 40 carbon atoms, a diarylamino group having 12 to 44 carbon atoms, a diaralkylamino group having 14 to 76 carbon atoms, an acyl group having 2 to 20 carbon atoms, an acyloxy group having 2 to 20 carbon atoms, an alkoxy group having 1 to 20 carbon atoms, an alkoxycarbonyl group having 2 to 20 carbon atoms, an alkoxycarbonyloxy group having 2 to 20 carbon atoms, or an alkylsulfonyl group having 1 to 20 carbon atoms . An alkyl group having 1 to 10 carbon atoms, an aralkyl group having 7 to 24 carbon atoms, an alkoxy group having 1 to 10 carbon atoms, or a diarylamino group having 12 to 36 carbon atoms is preferred. The number of the substituents is from 0 to 5, preferably from 0 to 2.

In this description, it is understood that the number of carbon atoms of a substituent is not included in the calculation of the number of carbon atoms. However, it is preferred that the above-mentioned number of carbon atoms be satisfied even when the number of carbon atoms of a substituent is included in the calculation.

Specific examples of the substituent include methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, icosyl, phenylmethyl, phenylethyl, phenylicosyl, naphthylmethyl, anthranylmethyl, phenanthrenylmethyl, pyrenylmethyl, vinyl, propenyl, butenyl, pentenyl, decenyl, icosenyl, ethynyl, propargyl, butynyl, pentynyl, decynyl, icosynyl, dimethylamino, ethylmethylamino, diethylamino, dipropylamino, dibutylamino, dipentynylamino, didecylamino, diicosylamino, diphenylamino, naphthylphenylamino, dinaphthylamino, dianthranylamino, diphenanthrenylamino, dipyrenylamino, diphenylmethylamino, diphenylethylamino, phenylmethylphenylethylamino, dinaphthylmethylamino, dianthranylmethylamino, diphenanthrenylmethylamino, acetyl, propionyl, butyryl, valeryl, benzoyl, acetyloxy, propionyloxy, butyryloxy, valeryloxy, benzoyloxy, methoxy, ethoxy, propoxy, butoxy, pentoxy, hexoxy, heptoxy, octoxy, nonyloxy, decyloxy, undecyloxy, dodecyloxy, tridecyloxy, tetradecyloxy, pentadecyloxy, hexadecyloxy, heptadecyloxy, octadecyloxy, nonadecyloxy, methoxycarbonyl, ethoxycarbonyl, propoxycarbonyl, butoxycarbonyl, pentoxycarbonyl, methoxycarbonyloxy, ethoxycarbonyloxy, propoxycarbonyloxy, butoxycarbonyloxy, pentoxycarbonyloxy, methylsulfonyl, ethylsulfonyl, propylsulfonyl, butylsulfonyl, and pentylsulfonyl. Of those, a C1 to C12 alkyl group, such as methyl, ethyl, propyl, butyl, pentyl, hexyl, heptyl, octyl, nonyl, or decyl, a C7 to C20 aralkyl group, such as phenylmethyl, phenylethyl, naphthylmethyl, anthranylmethyl, phenanthrenylmethyl, or pyrenylmethyl, a C1 to C10 alkoxy group, such as methoxy, ethoxy, propoxy, butoxy, pentoxy, hexoxy, heptoxy, octoxy, nonyloxy, or decyloxy, or a diarylamino group having two C6 to C15 aromatic hydrocarbon groups, such as diphenylamino, naphthylphenylamino, dinaphthylamino, dianthranylamino, or diphenanthrenylamino, is preferred.

R, R' , and R₁ to R₈ each independently represent hydrogen, an alkyl group having 1 to 20 carbon atoms, an aralkyl group having 7 to 38 carbon atoms, an alkenyl group having 2 to 20 carbon atoms, an alkynyl group having 2 to 20 carbon atoms, a dialkylamino group having 2 to 40 carbon atoms, a diarylamino group having 12 to 44 carbon atoms, a diaralkylamino group having 14 to 76 carbon atoms, an acyl group having 2 to 20 carbon atoms, an acyloxy group having 2 to 20 carbon atoms, an alkoxy group having 1 to 20 carbon atoms, an alkoxycarbonyl group having 2 to 20 carbon atoms, an alkoxycarbonyloxy group having 2 to 20 carbon atoms, an alkylsulfonyl group having 1 to 20 carbon atoms, an aromatic hydrocarbon group having 6 to 24 carbon atoms, or an aromatic heterocyclic group having 3 to 16 carbon atoms. Of those, hydrogen, an alkyl group having 1 to 10 carbon atoms, an aralkyl group having 7 to 24 carbon atoms, an alkoxy group having 1 to 10 carbon atoms, a diarylamino group having 12 to 36 carbon atoms, an aromatic hydrocarbon group having 6 to 18 carbon atoms, or an aromatic heterocyclic group having 3 to 16 carbonatoms ispreferred, and hydrogen, an aromatic hydrocarbon group having 6 to 18 carbon atoms, or an aromatic heterocyclic group having 3 to 16 carbon atoms is more preferred. In the case of the groups except hydrogen, the groups may each have a substituent.

Specific examples of the alkyl group having 1 to 20 carbon atoms, the aralkyl group having 7 to 38 carbon atoms, the alkenyl group having 2 to 20 carbon atoms, the alkynyl group having 2 to 20 carbon atoms, the dialkylamino group having 2 to 40 carbon atoms, the diarylamino group having 12 to 44 carbon atoms, the diaralkylamino group having 14 to 76 carbon atoms, the acyl group having 2 to 20 carbon atoms, the acyloxy group having 2 to 20 carbon atoms, the alkoxy group having 1 to 20 carbon atoms, the alkoxycarbonyl group having 2 to 20 carbon atoms, the alkoxycarbonyloxy group having 2 to 20 carbon atoms, or the alkylsulfonyl group having 1 to 20 carbon atoms include methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, icosyl, phenylmethyl, phenylethyl, phenylicosyl, naphthylmethyl, anthranylmethyl, phenanthrenylmethyl, pyrenylmethyl, vinyl, propenyl, butenyl, pentenyl, decenyl, icosenyl, ethynyl, propargyl, butynyl, pentynyl, decynyl, icosynyl, dimethylamino, ethylmethylamino, diethylamino, dipropylamino, dibutylamino, dipentynylamino, didecylamino, diicosylamino, diphenylamino, naphthylphenylamino, dinaphthylamino, dianthranylamino, diphenanthrenylamino, dipyrenylamino, diphenylmethylamino, diphenylethylamino, phenylmethylphenylethylamino, dinaphthylmethylamino, dianthranylmethylamino, diphenanthrenylmethylamino, acetyl, propionyl, butyryl, valeryl, benzoyl, acetyloxy, propionyloxy, butyryloxy, valeryloxy, benzoyloxy, methoxy, ethoxy, propoxy, butoxy, pentoxy, hexoxy, heptoxy, octoxy, nonyloxy, decyloxy, undecyloxy, dodecyloxy, tridecyloxy, tetradecyloxy, pentadecyloxy, hexadecyloxy, heptadecyloxy, octadecyloxy, nonadecyloxy, icosyloxy, methoxycarbonyl, ethoxycarbonyl, propoxycarbonyl, butoxycarbonyl, pentoxycarbonyl, methoxycarbonyloxy, ethoxycarbonyloxy, propoxycarbonyloxy, butoxycarbonyloxy, pentoxycarbonyloxy, methylsulfonyl, ethylsulfonyl, propylsulfonyl, butylsulfonyl, and pentylsulfonyl. Of those, an alkyl group having 1 to 10 carbon atoms, such as methyl, ethyl, propyl, butyl, pentyl, hexyl, heptyl, octyl, nonyl, or decyl, an aralkyl group having 7 to 17 carbon atoms, such as phenylmethyl, phenylethyl, naphthylmethyl, anthranylmethyl, phenanthrenylmethyl, or pyrenylmethyl, an alkoxy group having 1 to 10 carbon atoms, such as methoxy, ethoxy, propoxy, butoxy, pentoxy, hexoxy, heptoxy, octoxy, nonyloxy, or decyloxy, or a diarylamino group having 12 to 28 carbon atoms, such as diphenylamino, naphthylphenylamino, dinaphthylamino, dianthranylamino, or diphenanthrenylamino, is preferred.

Specific examples of the aromatic hydrocarbon group having 6 to 24 carbon atoms or the aromatic heterocyclic group having 3 to 16 carbon atoms include groups each produced by removing a hydrogen atom from benzene, pentalene, indene, naphthalene, azulene, indacene, acenaphthylene, phenalene, phenanthrene, anthracene, trindene, fluoranthene, acephenanthrylene, aceanthrylene, triphenylene, pyrene, chrysene, tetraphene, tetracene, pleiadene, picene, perylene, pentaphene, pentacene, tetraphenylene, cholanthrylene, furan, benzofuran, isobenzofuran, xanthene, oxanthrene, dibenzofuran, peri-xanthenoxanthene, thiophene, thioxanthene, thianthrene, phenoxathiin, thionaphthene, isothianaphthene, thiophthene, thiophanthrene, dibenzothiophene, pyrrole,pyrazole, tellurazole, selenazole, thiazole, isothiazole, oxazole, furazan, thiadiazole, pyridine, pyrazine, pyrimidine, pyridazine, triazine, indolizine, indole, isoindole, indazole, purine, quinolizine, isoquinoline, carbazole, imidazole, naphthyridine, phthalazine, quinazoline, benzodiazepine, quinoxaline, cinnoline, quinoline, pteridine, phenanthridine, acridine, perimidine, phenanthroline, phenazine, carboline, phenotellurazine, phenoselenazine, phenothiazine, phenoxazine, anthyridine, benzothiazole, benzimidazole, benzoxazole, benzisoxazole, or benzisothiazole. Of those, a group produced by removing a hydrogen atom from benzene, naphthalene, anthracene, pyridine, pyrazine, pyrimidine, pyridazine, triazine, isoindole, indazole, purine, isoquinoline, imidazole, naphthyridine, phthalazine, quinazoline, benzodiazepine, quinoxaline, cinnoline, quinoline, pteridine, phenanthridine, acridine, perimidine, phenanthroline, phenazine, carboline, indole, carbazole, dibenzofuran, or dibenzothiophene is preferred.

When R, R', and R₁ to R₈ described above each represent a group except hydrogen, and the group has a substituent, the substituent is an alkyl group having 1 to 20 carbon atoms, an aralkyl group having 7 to 38 carbon atoms, an alkenyl group having 2 to 20 carbon atoms, an alkynyl group having 2 to 20 carbon atoms, a dialkylamino group having 2 to 40 carbon atoms, a diarylamino group having 12 to 44 carbon atoms, a diaralkylamino group having 14 to 76 carbon atoms, an acyl group having 2 to 20 carbon atoms, an acyloxy group having 2 to 20 carbon atoms, an alkoxy group having 1 to 20 carbon atoms, an alkoxycarbonyl group having 2 to 20 carbon atoms, an alkoxycarbonyloxy group having 2 to 20 carbon atoms, an alkylsulfonyl group having 1 to 20 carbon atoms, an aromatic hydrocarbon group having 6 to 24 carbon atoms, or an aromatic heterocyclic group having 3 to 16 carbon atoms . An alkyl group having 1 to 10 carbon atoms, an aralkyl group having 7 to 24 carbon atoms, an alkoxy group having 1 to 10 carbon atoms, a diarylamino group having 12 to 36 carbon atoms, an aromatic hydrocarbon group having 6 to 18 carbon atoms, or an aromatic heterocyclic group having 3 to 16 carbon atoms is preferred, and an aromatic hydrocarbon group having 6 to 18 carbon atoms, or an aromatic heterocyclic group having 3 to 16 carbon atoms is more preferred. The number of the substituents per one of R, R' , and R₁ to R₈ is preferably from 0 to 3, more preferably from 0 to 2.

Specific examples of the alkyl group having 1 to 20 carbon atoms, the aralkyl group having 7 to 38 carbon atoms, the alkenyl group having 2 to 20 carbon atoms, the alkynyl group having 2 to 20 carbon atoms, the dialkylamino group having 2 to 40 carbon atoms, the diarylamino group having 12 to 44 carbon atoms, the diaralkylamino group having 14 to 76 carbon atoms, the acyl group having 2 to 20 carbon atoms, the acyloxy group having 2 to 20 carbon atoms, the alkoxy group having 1 to 20 carbon atoms, the alkoxycarbonyl group having 2 to 20 carbon atoms, the alkoxycarbonyloxy group having 2 to 20 carbon atoms, the alkylsulfonyl group having 1 to 20 carbon atoms, the aromatic hydrocarbon group having 6 to 24 carbon atoms, and the aromatic heterocyclic group having 3 to 16 carbon atoms are same as those described in the specific examples of R, R', and R₁ to Re described in the foregoing.

Preferred specific examples of the compounds represented by the general formulae (1) and (2) are shown below, but the compounds are not limited thereto.

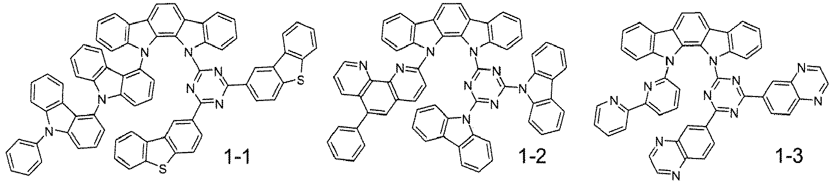

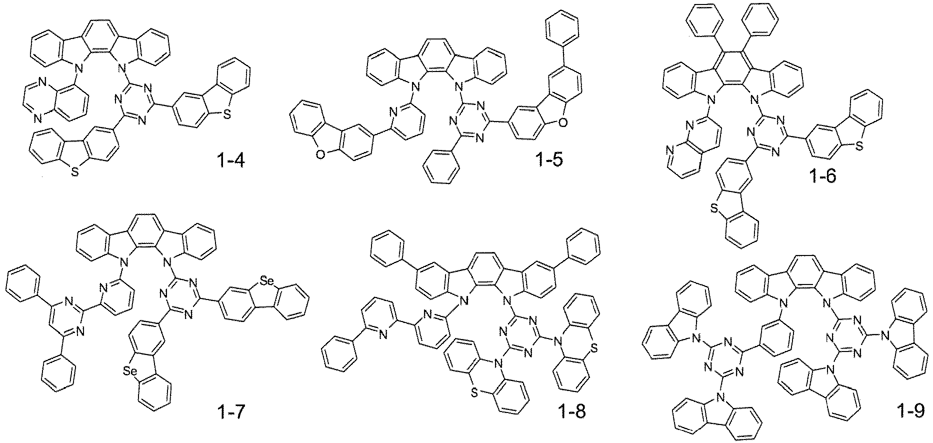

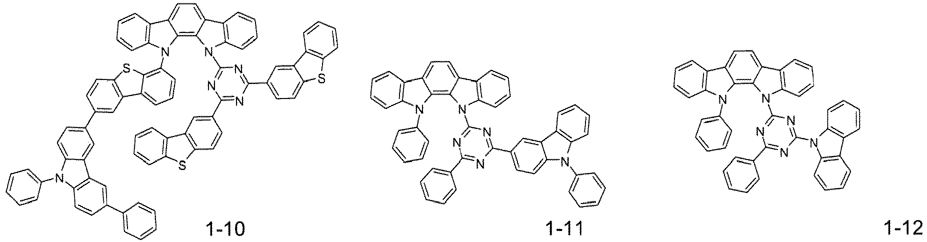

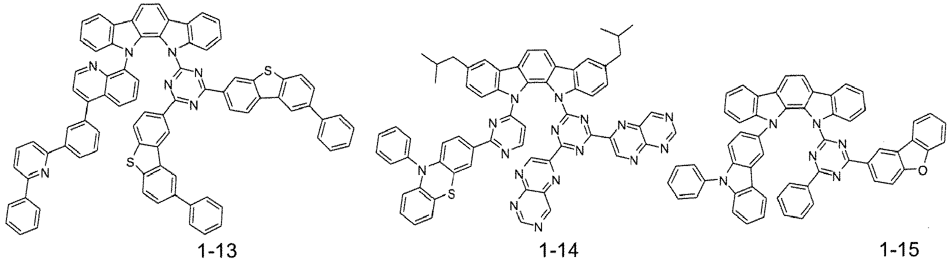

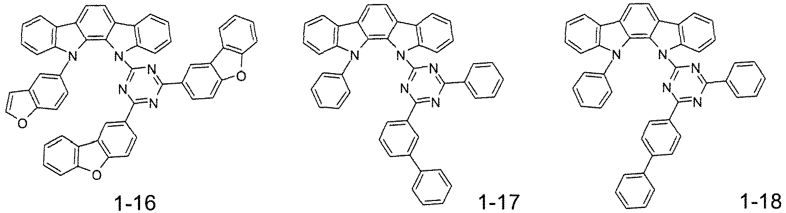

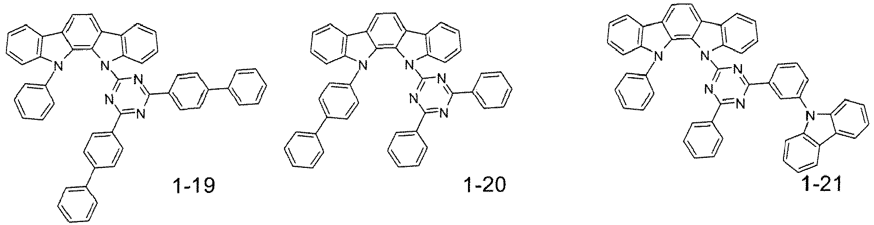

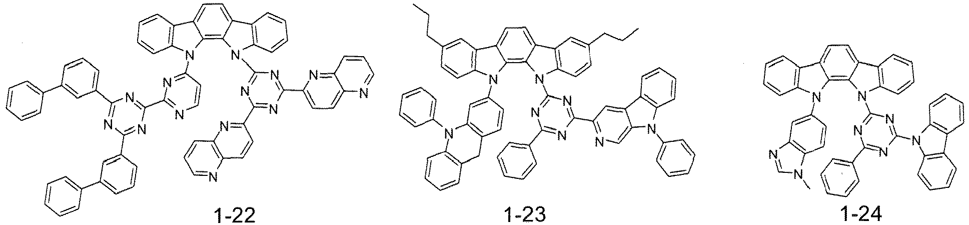

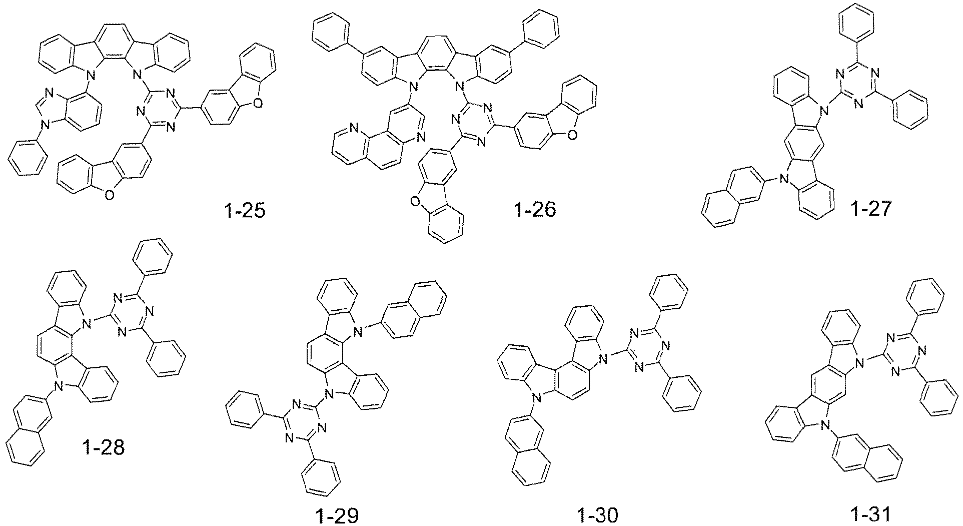

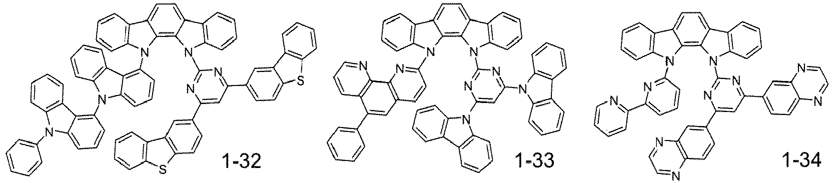

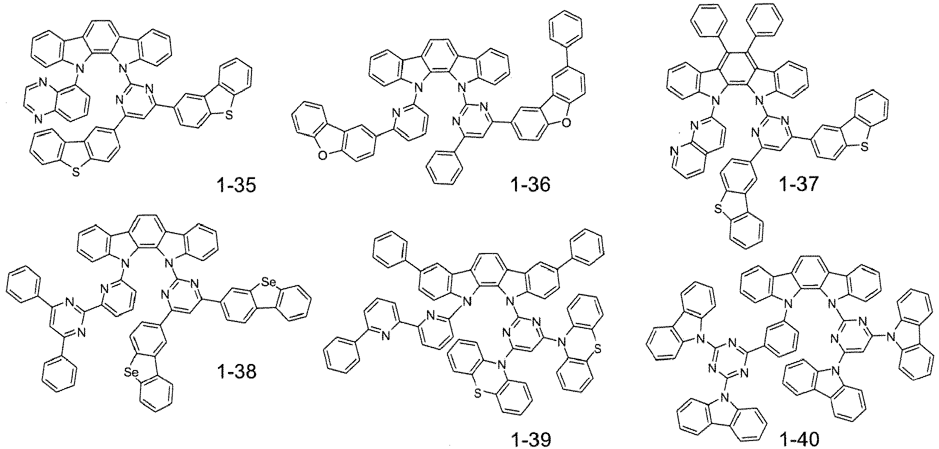

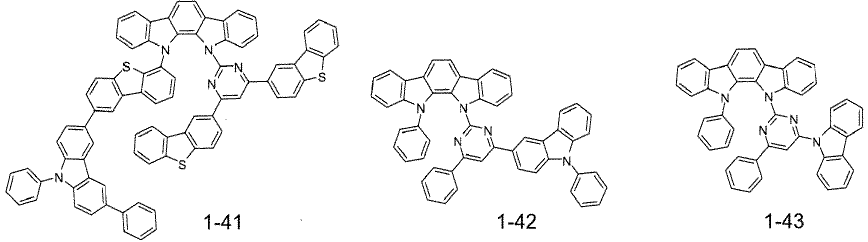

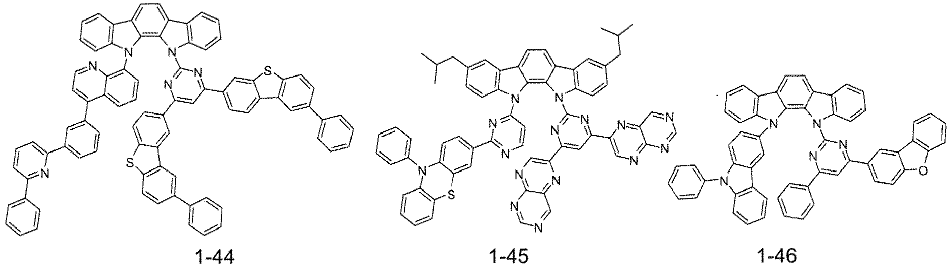

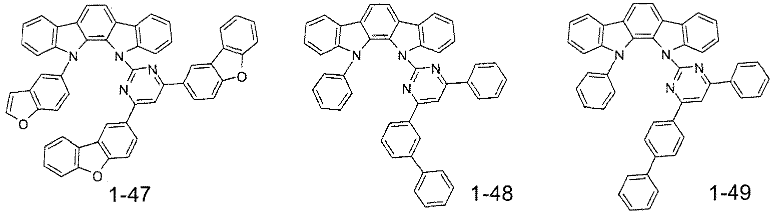

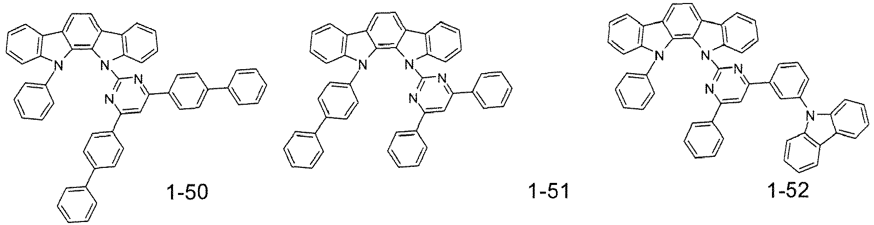

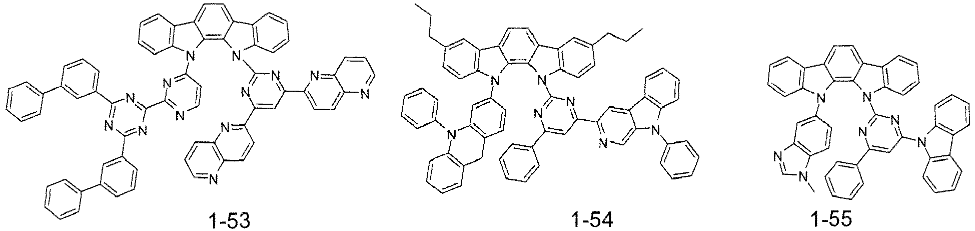

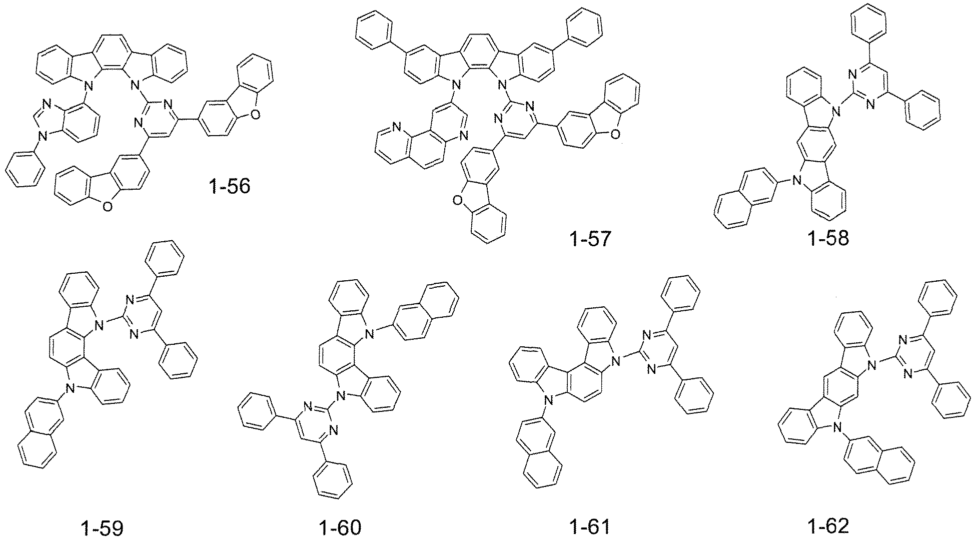

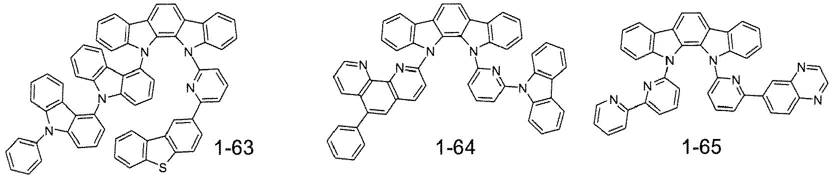

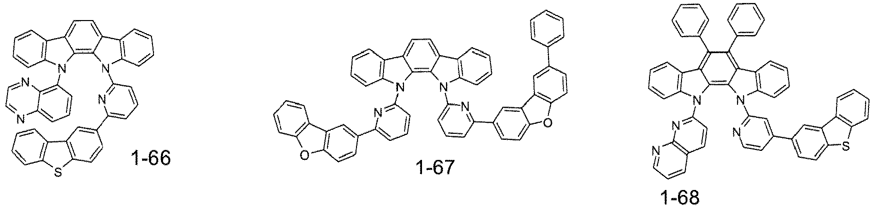

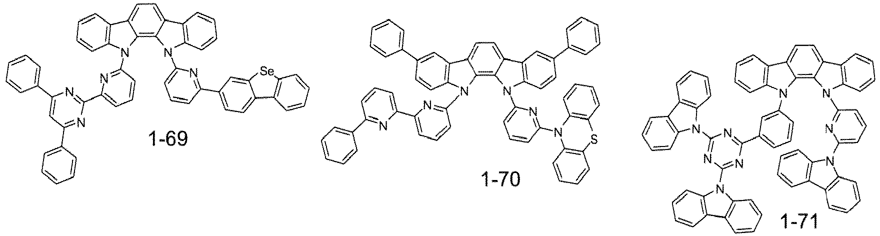

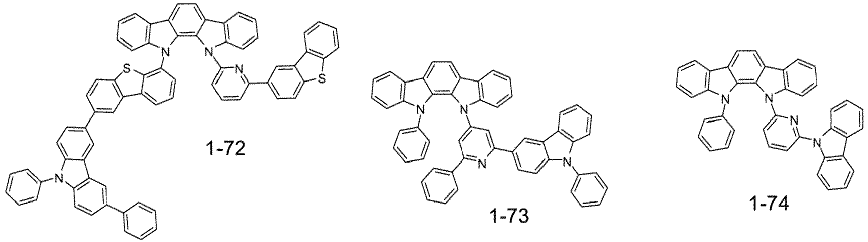

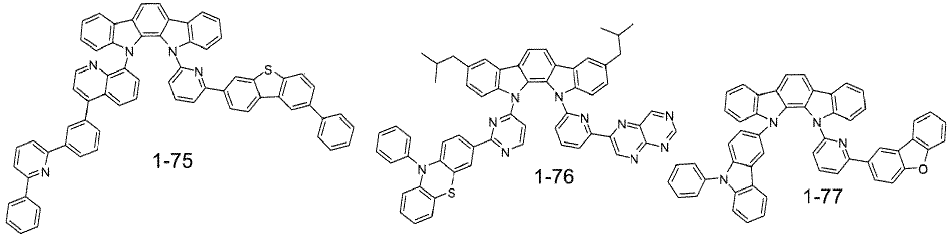

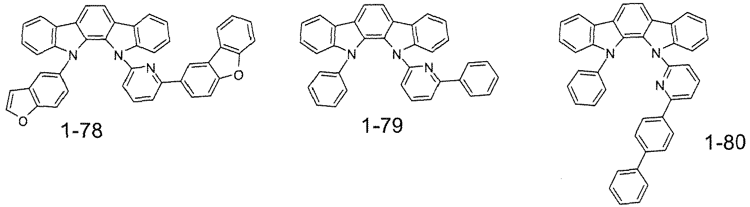

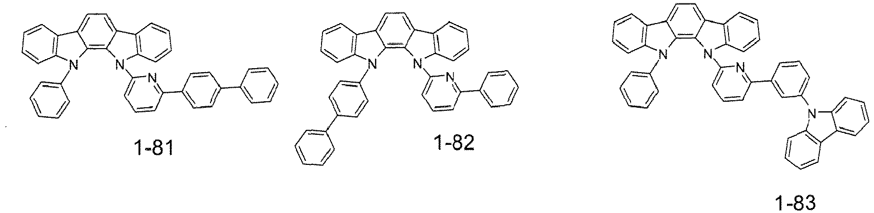

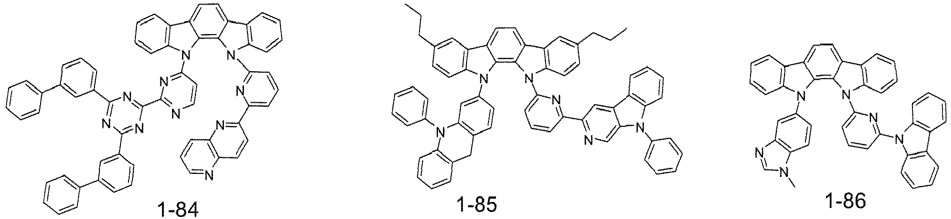

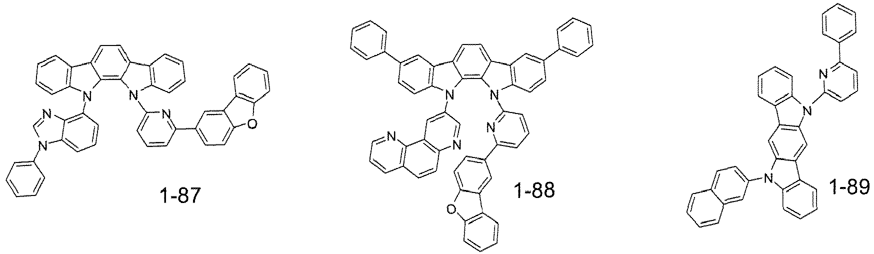

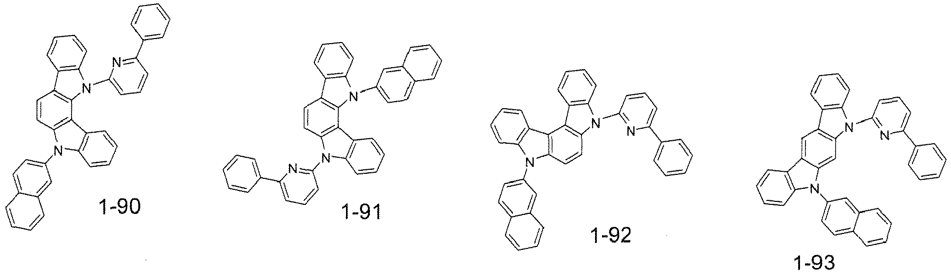

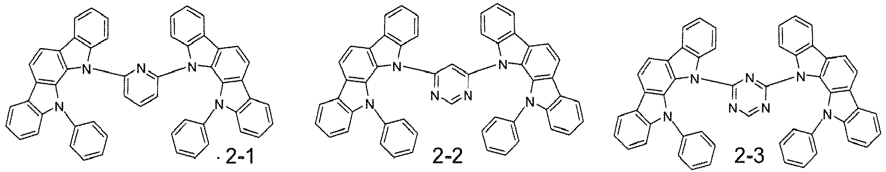

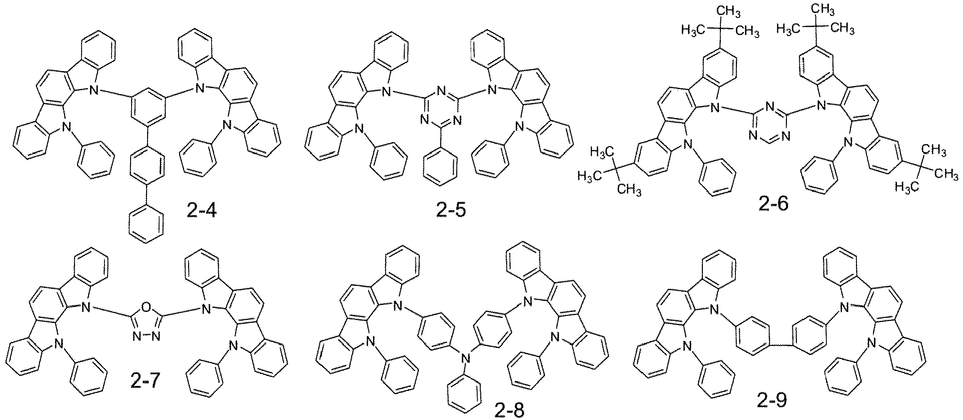

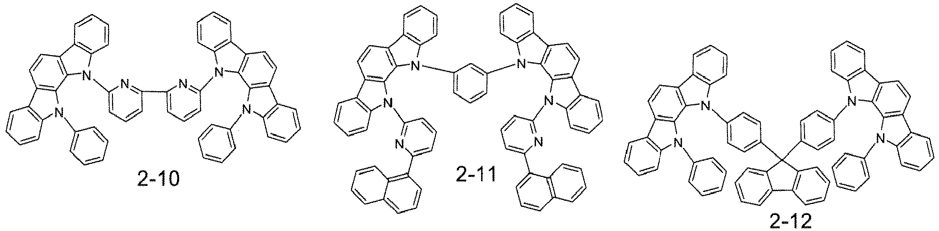

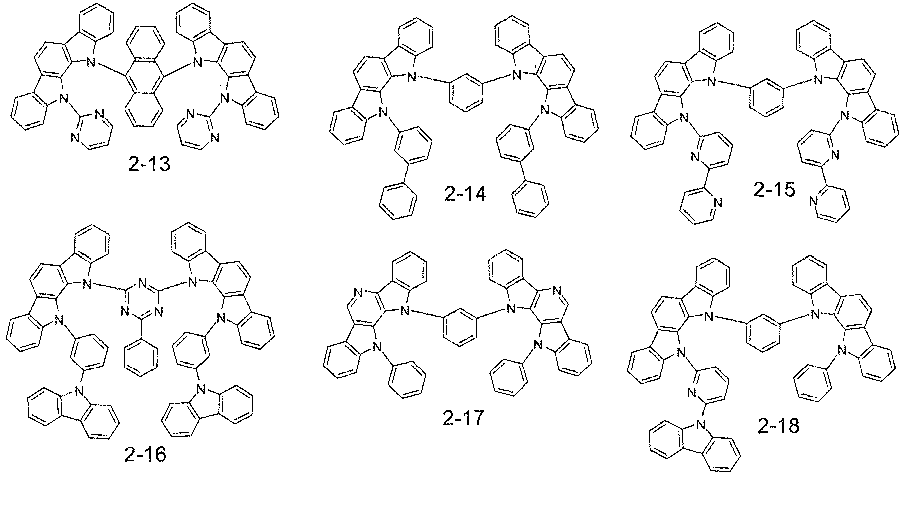

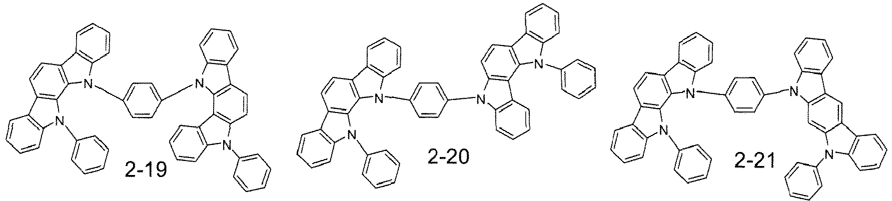

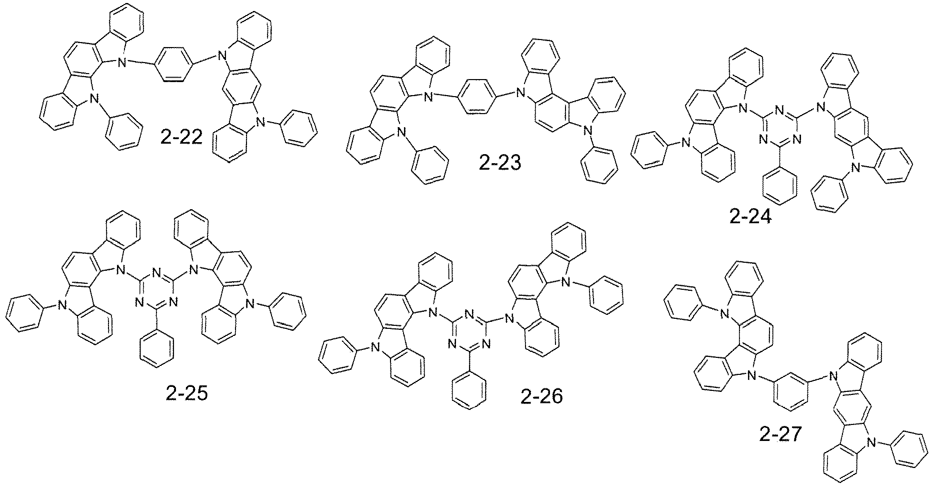

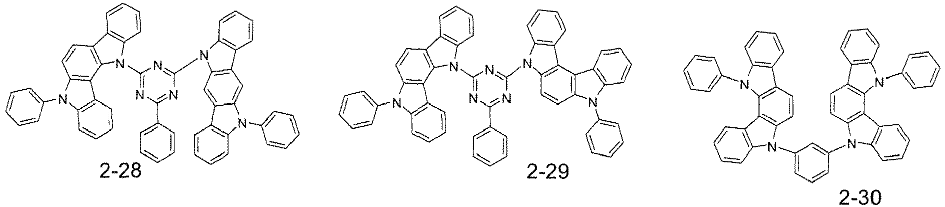

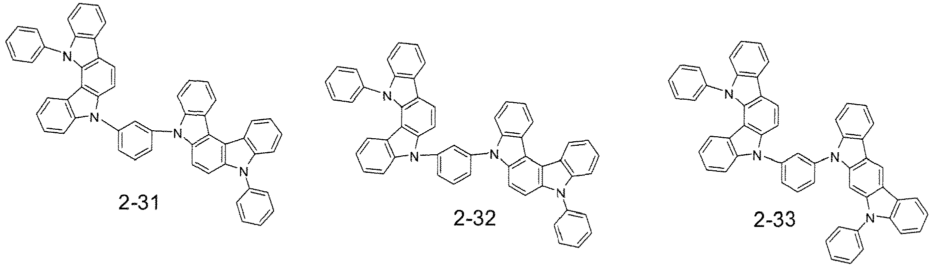

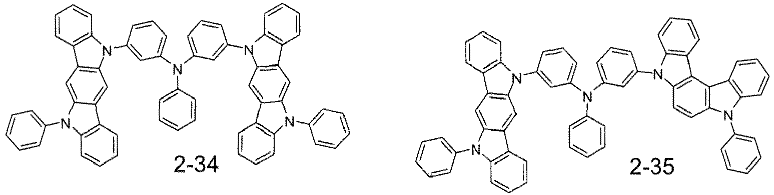

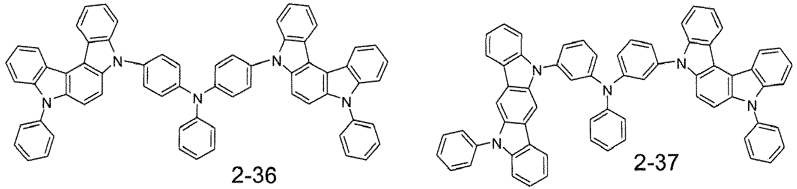

Next, the general formula (3) is described. j represents an integer of 1 or 3, preferably an integer of 1.

R₉ to R₁₂ each independently represent an aromatic hydrocarbon group having 6 to 18 carbon atoms, or an aromatic heterocyclic group having 3 to 9 carbon atoms.

Ar₄ represents hydrogen or an aromatic hydrocarbon group having 6 to 24 carbon atoms. In addition, at least one Ar₄ represents a group except hydrogen, i.e., a monovalent aromatic hydrocarbon group or aromatic heterocyclic group.

Specific examples of the aromatic hydrocarbon group represented by Ar₄ are the same as those described in the specific examples of L₁ and L₂ described in the foregoing.

k, l, m, and n represent integers satisfying 0≤k+l+m+n≤16, preferably 0≤k+l+m+n≤4. k, 1, m, and n each represent preferably from 0 to 2, more preferably 0 or 1.

X₃ to X₅ each independently represent N, C-R", or C-. R"'s each independently represent hydrogen, an alkyl group having 1 to 20 carbon atoms, an alkoxy group having 1 to 20 carbon atoms, or a diarylamino group having 12 to 44 carbon atoms, preferably hydrogen, an alkyl group having 1 to 10 carbon atoms, an alkoxy group having 1 to 10 carbon atoms, or a diarylamino group having 12 to 36 carbon atoms, more preferably hydrogen, an alkyl group having 1 to 10 carbon atoms, or an alkoxy group having 1 to 10 carbon atoms. Specific examples of R" are the same as the corresponding specific examples described for R, R', and R₁ to R₈ described in the general formulae (1) and (2).

Preferred specific examples of the compound represented by the general formula (3) are
compounds 3-2, 3-9, 3-11 to 3-14, 3-16, 3-18, 3-24, 3-28 to 3-31, 3-35, and 3-37 to 3-38.

Note that compounds 3-1, 3-3 to 3-8, 3-10, 3-15, 3-17, 3-19 to 3-23, 3-25 to 3-27, 3-32 to 3-34, 3-36, and 3-39 to 3-54 are not according to the claimed invention.

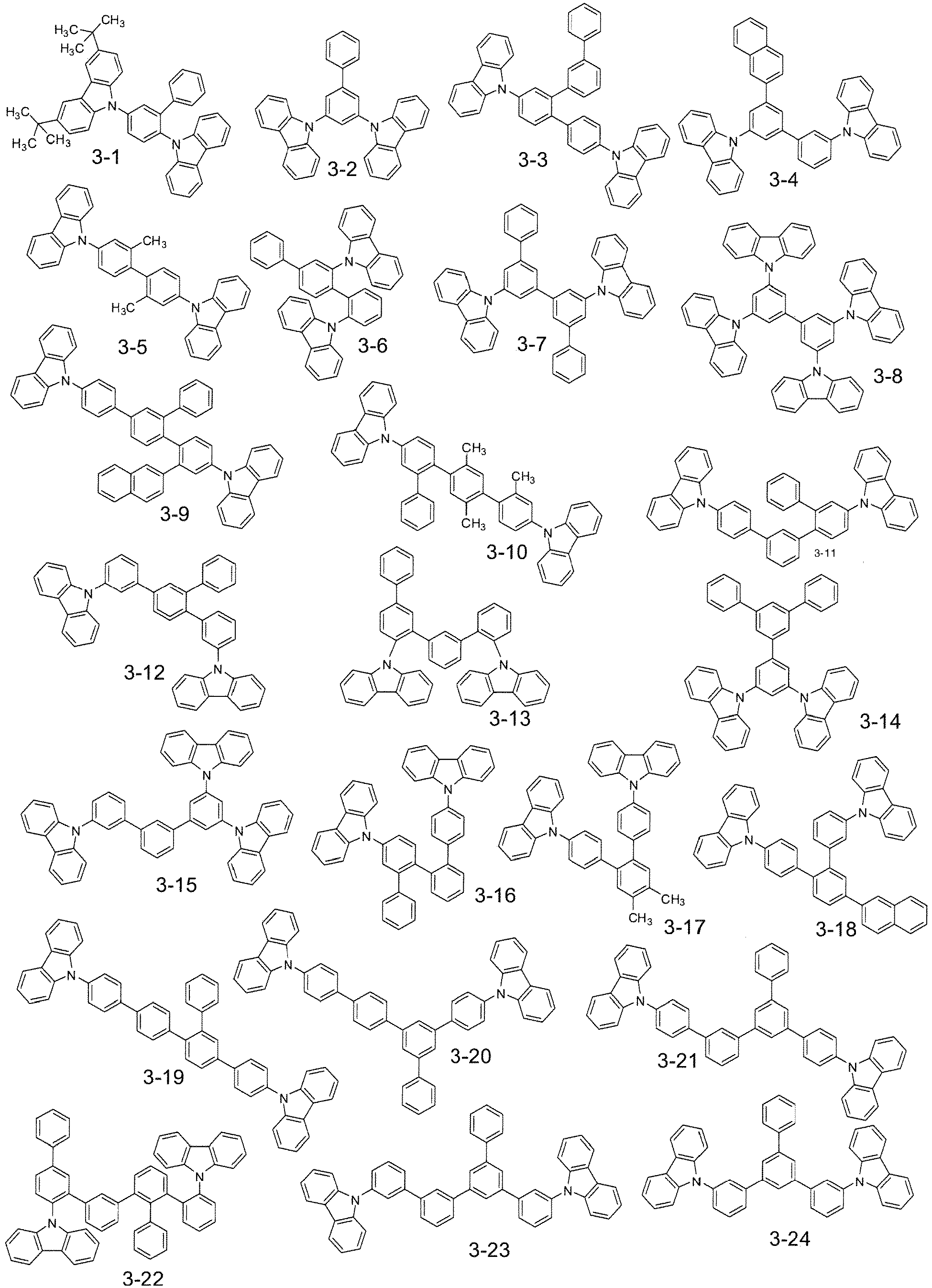

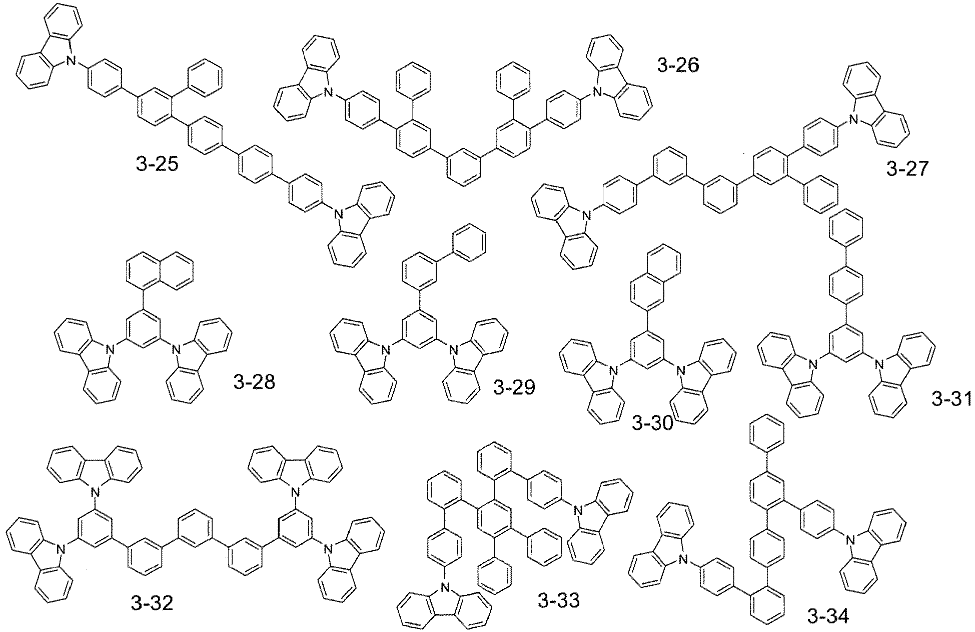

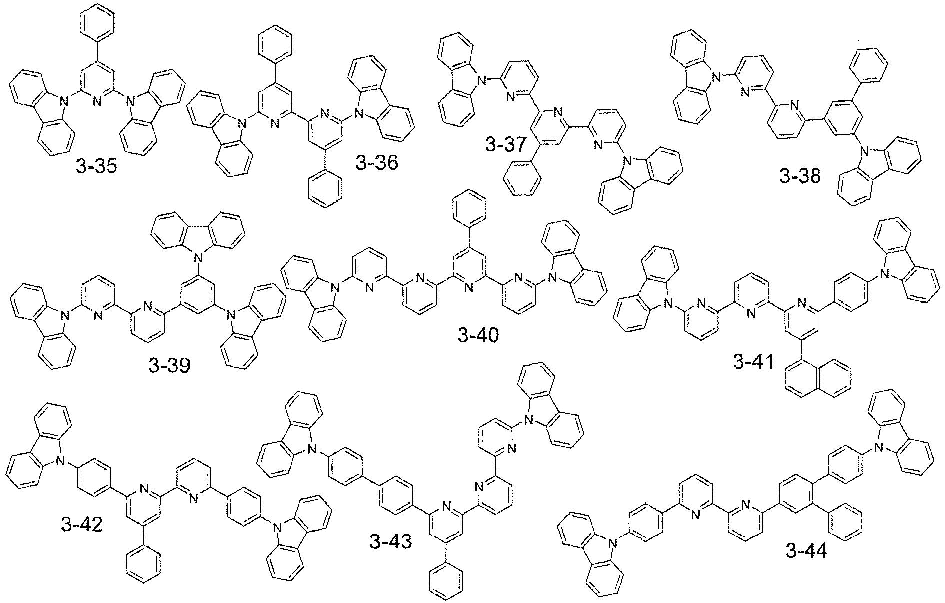

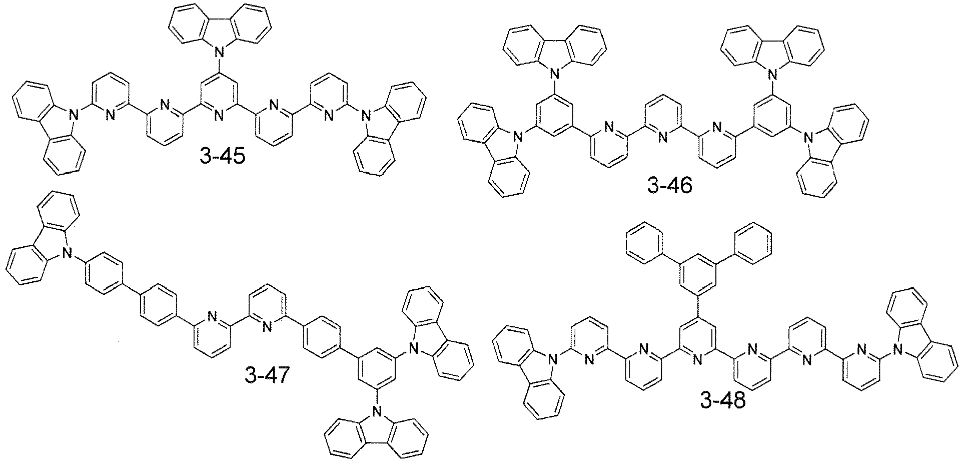

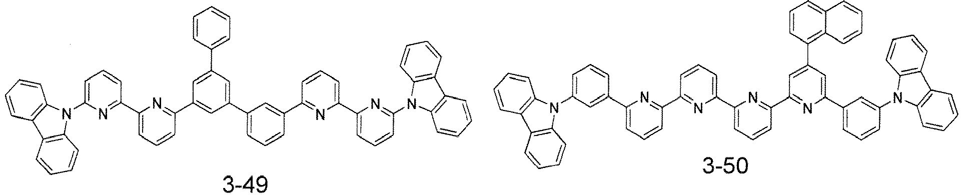

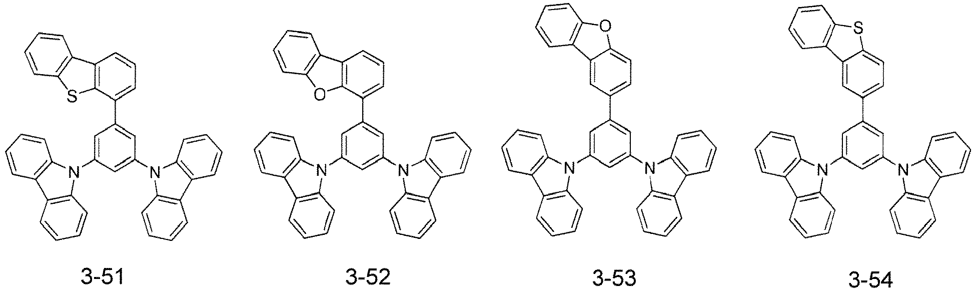

Next, the first host material (H1) and the second host material (H2) are described.
When an EA difference (ΔEA) between the first host material (H1) and the second host material (H2) is more than 0.1 eV, a satisfactory result is obtained. When hosts each having a ΔEA of 0.1 eV or less are mixed, charge balance remains substantially unchanged, and hence thin film stability can be improved without the impairment of original device characteristics. However, when hosts each having a ΔEA of more than 0.1 eV are mixed, in contrast, the path along which an electron flows can be limited to the host having the larger EA out of the two hosts to be mixed, and hence the flow of an electron in the light-emitting layer can be suppressed. As a result, an electron can be easily confined in the light-emitting layer, and hence a device having a long lifetime while having high efficiency can be provided. The ΔEA desirably falls within the range of from 0.2 eV to 1.5 eV. A value for an EA can be calculated by using: a value for an ionization potential obtained by photoelectron spectroscopy in a host material thin film; and a value for an energy gap determined from an absorption edge of a measured UV-visible absorption spectrum. However, a measurement method is not limited thereto. When one, or each of both, of the first host material (H1) and the second host material (H2) contains two or more host materials, the EA difference is calculated by using a host material that is most abundant in the first host material (H1) or the second host material (H2).

The two host materials including the first host material (H1) and the second host material (H2) may be mixed before the production of the device and vapor-deposited by using one vapor deposition source, or may be mixed at the time of the production of the device by an operation such as co-deposition involving using a plurality of vapor deposition sources. A mixing ratio (weight ratio) between the host materials, which is not particularly limited, falls within preferably the range of from 95:5 to 5:95, more preferably the range of 90:10 to 10:90. Although a host material that does not correspond to any of the general formulae (1), (2), and (3) can be used as a host material to such an extent that the effects of the present invention are not impaired, its usage amount is desirably limited to 30 wt% or less, preferably 10 wt% or less.

In addition, a presence ratio of each of the first host material (H1) and the second host material (H2) in the light-emitting layer desirably changes by an amount within 5% relative to a preliminary mixing ratio of each of the materials before the vapor deposition. To this end, it is advantageous to uniformize the vaporization rates of both the materials to a certain range. The vaporization rates relate to vapor pressures at the time of the evaporation or sublimation of those materials, and relate to vaporization temperatures (temperatures at which the vapor pressures coincide with pressures at the time of the vapor deposition). Accordingly, a difference in vaporization temperature between both the materials is desirably set to within 30°C, preferably within 10°C.

Next, the structure of the organic EL device of the present invention is described with reference to the drawings. However, the structure of the organic EL device of the present invention is by no means limited to one illustrated in the drawings.

### (1) Construction of Organic EL Device

FIG. 1 is a sectional view for schematically illustrating a structure example of a general organic EL device to be used in the present invention. Reference numeral 1 represents a substrate, reference numeral 2 represents an anode, reference numeral 3 represents a hole-injecting layer, reference numeral 4 represents a hole-transporting layer, reference numeral 5 represents a light-emitting layer, reference numeral 6 represents an electron-transporting layer, reference numeral 7 represents an electron-injecting layer, and reference numeral 8 represents a cathode. The organic EL device of the present invention includes the anode, the light-emitting layer, the electron-transporting layer, and the cathode as its essential layers, and may include any other layer as required. Examples of the other layer include, but not limited to, a hole-injecting/transporting layer, an electron-blocking layer, and a hole-blocking layer. The hole-injecting/transporting layer means any one or both of the hole-injecting layer and the hole-transporting layer.

### (2) Substrate

The substrate 1 serves as a support for the organic electroluminescent device, and a quartz or glass plate, a metal plate or a metal foil, a plastic film or sheet, or the like is used. A glass plate, or a smooth and transparent plate made of a synthetic resin, such as polyester, polymethacrylate, polycarbonate, or polysulfone, is particularly preferred. When a synthetic resin substrate is used, attention needs to be paid to its gas barrier property. The case in which the gas barrier property of the substrate is excessively small is not preferred because the organic electroluminescent device may deteriorate owing to outside air that has passed the substrate. Accordingly, a method involving providing at least one surface of the synthetic resin substrate with a dense silicon oxide film or the like to secure the gas barrier property is one preferred method.

### (3) Anode

The anode 2 is formed on the substrate 1 and the anode serves to inject a hole into the hole-transporting layer. The anode is typically formed of, for example, a metal, such as aluminum, gold, silver, nickel, palladium, or platinum, a metal oxide, such as an oxide of indium and/or tin, or an oxide of indium and/or zinc, a metal halide, such as copper iodide, carbon black, or a conductive polymer, such as poly(3-methylthiophene), polypyrrole, or polyaniline. The formation of the anode is typically performed by, for example, a sputtering method or a vacuum deposition method in many cases. In addition, in the case of, for example, a metal fine particle made of silver or the like, a fine particle made of copper iodide or the like, carbon black, a conductive metal oxide fine particle, or conductive polymer fine powder, the anode can be formed by dispersing such particle or powder in a proper binder resin solution and applying the dispersion onto the substrate. Further, in the case of a conductive polymer, the anode can be formed by directly forming a thin film of the conductive polymer on the substrate through electrolytic polymerization or by applying the conductive polymer onto the substrate 1. The anode can also be formed by laminating different substances. The thickness of the anode varies depending on transparency to be required. When the transparency is required, the visible light transmittance of the anode is desirably set to 60% or more, preferably 80% or more in ordinary cases. In such cases, the thickness is typically from about 5 nm to about 1,000 nm, preferably from about 10 nm to about 500 nm. When the anode may be opaque, the anode may have the same transmittance as that of the substrate. In addition, another conductive material can be further laminated on the anode.

### (4) Hole-transporting Layer

The hole-transporting layer 4 is formed on the anode 2. The hole-injecting layer 3 can be formed therebetween. A material for the hole-transporting layer is required to satisfy the following conditions: the material needs to have high efficiency with which a hole is injected from the anode and be capable of efficiently transporting the inj ectedhole . To this end, the material is required to have a small ionization potential, have high transparency for visible light, have a large hole mobility, be excellent in stability, and hardly produce an impurity serving as a trap at the time of the production or use. In addition, the layer is in contact with the light-emitting layer 5, and is hence required neither to quench light emitted from the light-emitting layer nor to form an exciplex between itself and the light-emitting layer to reduce the efficiency. In addition to the above-mentioned general requirements, the device is required to further have heat resistance when its application to an on-vehicle display is considered. Therefore, a material having a Tg of 85°C or more is desired.

A known compound that has heretofore been used in the layer can be used as a hole-transporting material that can be used in the present invention. Examples thereof include: an aromatic diamine which contains two or more tertiary amines and in which a nitrogen atom is substituted with two or more fused aromatic rings ; an aromatic amine compound having a starburst structure, such as 4,4',4"-tris(1-naphthylphenylamino)triphenylamine; an aromatic amine compound formed of a tetramer of triphenylamine; and a spiro compound, such as 2,2',7,7'-tetrakis-(diphenylamino)-9,9'-spirobifluorene. Those compounds may be used alone or as a mixture thereof as required.

In addition, examples of the material for the hole-transporting layer other than the above-mentioned compounds include polymer materials, such as polyvinylcarbazole, polyvinyltriphenylamine, and tetraphenylbenzidine-containing polyarylene ether sulfone.

When the hole-transporting layer is formed by an application method, the hole-transporting layer is formed by: adding and dissolving one or two or more kinds of hole-transporting materials, and as required, an additive that does not serve as a trap for a hole, such as a binder resin or an applicability improver, to prepare an application solution; applying the solution onto the anode by a method such as a spin coating method; and drying the applied solution. Examples of the binder resin include polycarbonate, polyarylate, and polyester. When the binder resin is added in a large amount, a hole mobility reduces. Accordingly, the addition amount is desirably as small as possible and is preferably 50 wt% or less in ordinary cases.

When the hole-transporting layer is formed by the vacuum deposition method, the hole-transporting layer is formed by: loading a hole-transporting material into a crucible placed in a vacuum chamber; evacuating the inside of the vacuum chamber to about 10⁻⁴ Pa with a proper vacuum pump; and heating the crucible after the evacuation to evaporate the hole-transporting material. Thus, the hole-transporting layer is formed on the substrate having formed thereon the anode, the substrate being placed to face the crucible. The thickness of the hole-transporting layer is typically from 1 nm to 300 nm, preferably from 5 nm to 100 nm. In general, the vacuum deposition method is frequently employed for uniformly forming such thin film.

### (5) Hole-injecting Layer

The hole-injecting layer 3 has been inserted between the hole-transporting layer 4 and the anode 2 for the purposes of additionally improving the hole injection efficiency and improving the adhesive force of the entire organic layer to the anode. The insertion of the hole-injecting layer provides the following effects: the initial driving voltage of the device reduces, and at the same time, an increase in voltage when the device is continuously driven at a constant current is suppressed. A material to be used in the hole-injecting layer is required to satisfy the following conditions: the material can be formed into a uniform thin film, which can be satisfactorily brought into contact with the anode, and is thermally stable, i.e., has a high glass transition temperature. The material is required to have a glass transition temperature of 100°C or more. Further, the material is required to satisfy, for example, the following conditions: the material has a low ionization potential and hence facilitates the injection of a hole from the anode; and the material has a large hole mobility.

To this end, the following materials have been reported hitherto: a phthalocyanine compound, such as copper phthalocyanine, an organic compound, such as polyaniline or polythiophene, a sputtered carbon film, a metal oxide, such as a vanadium oxide, a ruthenium oxide, or a molybdenum oxide, and a P-type organic substance, such as 1, 4,5, 8 -naphthalenetetracarboxylic dianhydride (NTCDA) or hexanitrilehexaazatriphenylene (HAT). Those compounds may be used alone or as a mixture thereof as required. A thin film serving as the hole-injecting layer can be formed as in the hole-transporting layer. In the case of an inorganic substance, however, the sputtering method, an electron beam deposition method, or a plasma CVD method is further employed. The thickness of the hole-injecting layer to be formed as described above is typically from 1 nm to 300 nm, preferably from 5 nm to 100 nm.

### (6) Light-emitting Layer

The light-emitting layer 5 is formed on the hole-transporting layer 4. The light-emitting layer may be formed of a single light-emitting layer, or may be formed by laminating a plurality of light-emitting layers so that the layers may be in direct contact with each other. The light-emitting layer includes the two host materials, i.e., the first host material (H1) and the second host material (H2), and a fluorescent light-emitting material or a phosphorescent light-emitting material. The two host materials are desirably a combination of a compound represented by the general formula (1) or (2), and a compound represented by any one of the general formulae (1) to (3), particularly desirably a combination of a compound represented by the general formula (1) or (2), and a compound represented by the general formula (3).

A fused ring derivative, such as perylene or rubrene, a quinacridone derivative, phenoxazone 660, DCM1, perinone, a coumarin derivative, a pyrromethene (diazaindacene) derivative, a cyanine dye, or the like can be used as the fluorescent light-emitting material to be added to the host material.

When a fluorescent light-emitting dopant is used as the light-emitting dopant material, the fluorescent light-emitting dopant is not particularly limited, and examples thereof include a benzoxazole derivative, a benzothiazole derivative, a benzimidazole derivative, a styrylbenzene derivative, a polyphenyl derivative, a diphenylbutadiene derivative, a tetraphenylbutadiene derivative, a naphthalimide derivative, a coumarin derivative, a fused aromatic compound, a perinone derivative, an oxadiazole derivative, an oxazine derivative, an aldazine derivative, a pyrrolidine derivative, a cyclopentadiene derivative, a bisstyrylanthracene derivative, a quinacridone derivative, a pyrrolopyridine derivative, a thiadiazolopyridine derivative, a styrylamine derivative, a diketopyrrolopyrrole derivative, an aromatic dimethylidyne compound, various metal complexes typified by a metal complex of an 8-quinolinol derivative, a metal complex, rare earth complex, or transition metal complex of a pyrromethene derivative, polymer compounds, such as polythiophene, polyphenylene, and polyphenylene vinylene, and an organic silane derivative. Of those, the following compound is preferred: a fused aromatic derivative, a styryl derivative, a diketopyrrolopyrrole derivative, an oxazine derivative, a pyrromethene metal complex, transition metal complex, or lanthanoid complex. For example, the following compound is more preferred: naphthacene, pyrene, chrysene, triphenylene, benzo[c]phenanthrene, benzo [a] anthracene, pentacene, perylene, fluoranthene, acenaphthofluoranthene, dibenzo[a,j]anthracene, dibenzo[a,h]anthracene, benzo[a]naphthacene, hexacene, naphtho[2,1-f]isoquinoline, α-naphthaphenanthridine, phenanthroxazole, quinolino[6,5-f]quinoline, or benzothiophanthrene. Those compounds may each have an alkyl group, an aryl group, an aromatic heterocyclic group, or a diarylamino group as a substituent.

Only one kind of fluorescent light-emitting dopant material may be incorporated into the light-emitting layer, or two or more kinds of fluorescent light-emitting dopant materials may be incorporated into the layer. When two or more kinds of fluorescent light-emitting dopant materials are incorporated into the layer, the total weight of the fluorescent light-emitting dopant materials is preferably 20% or less, more preferably 10% or less with respect to the host materials.

When a thermally activated delayed fluorescent light-emitting dopant is used as the organic light-emitting dopant material, the thermally activated delayed fluorescent light-emitting dopant is not particularly limited, and examples thereof include: metal complexes, such as a tin complex and a copper complex; indolocarbazole derivatives disclosed in WO 2011/070963 A1; cyanobenzene derivatives and carbazole derivatives disclosed in Nature 2012, 492, 234; and phenazine derivatives, oxadiazole derivatives, triazole derivatives, sulfone derivatives, phenoxazine derivatives, and acridine derivatives disclosed in Nature Photonics 2014, 8, 326.

The thermally activated delayed fluorescent light-emitting dopant material is not particularly limited, and specific examples thereof include the following compounds.

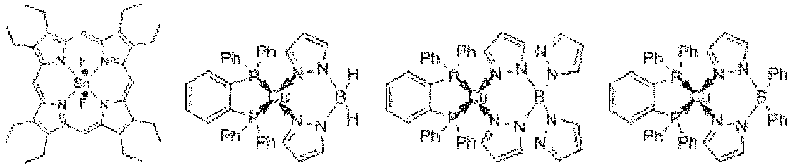

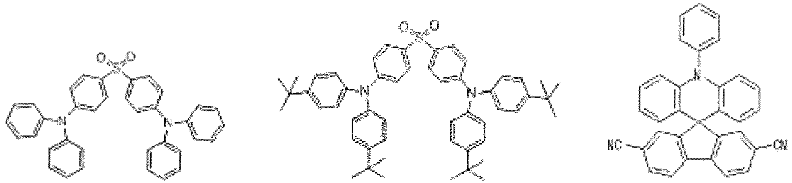

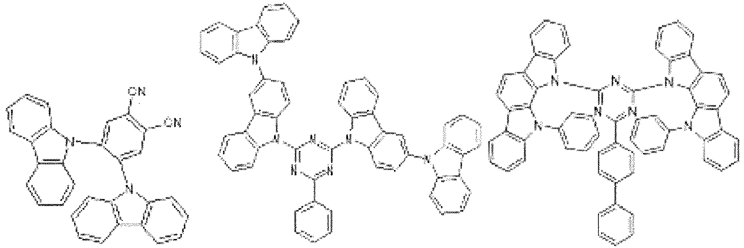

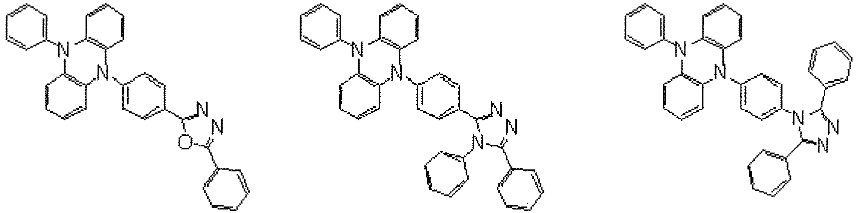

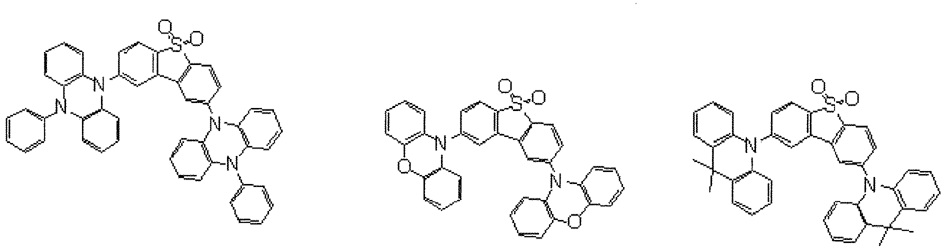

Only one kind of thermally activated delayed fluorescent light-emitting dopant material may be incorporated into the light-emitting layer, or two or more kinds of thermally activated delayed fluorescent light-emitting dopant materials may be incorporated into the layer. In addition, the thermally activated delayed fluorescent light-emitting dopant material maybe used after having been mixed with a phosphorescent light-emitting dopant or a fluorescent light-emitting dopant. When two or more kinds of light-emitting dopant materials including the thermally activated delayed fluorescent light-emitting dopant material are incorporated into the layer, the total weight of the light-emitting dopant materials is preferably 50% or less, more preferably 30% or less with respect to the host materials.

The phosphorescent light-emitting material to be added to the host materials desirably contains an organometallic complex containing at least one metal selected from, for example, ruthenium, rhodium, palladium, silver, rhenium, osmium, iridium, platinum, and gold. Specific examples thereof include, but not limited to, the compounds disclosed in the following literatures.

WO 2009-073245 A1, WO 2009-046266 A1, WO 2007-095118 A2, WO 2008-156879 A1, WO 2008-140657 A1, US 2008/261076 A, JP 2008-542203 A, WO 2008-054584 A1, JP 2008-505925 A, JP 2007-522126 A, JP 2004-506305 A, and the like.

Preferred examples of the phosphorescent light-emitting dopant include complexes such as Ir(ppy)3, complexes such as Ir (bt) 2 · acac3 , and complexes such as PtOEt3, the complexes each having a noble metal element such as Ir as a central metal. Specific examples of those complexes are shown below, but the complexes are not limited to the following compounds.

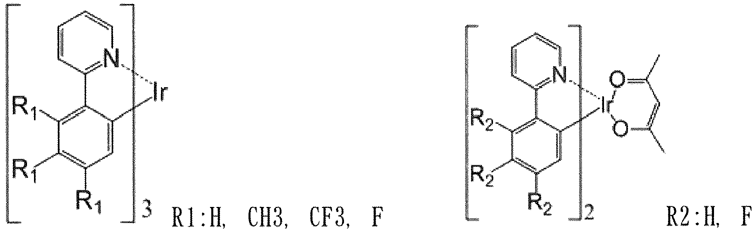

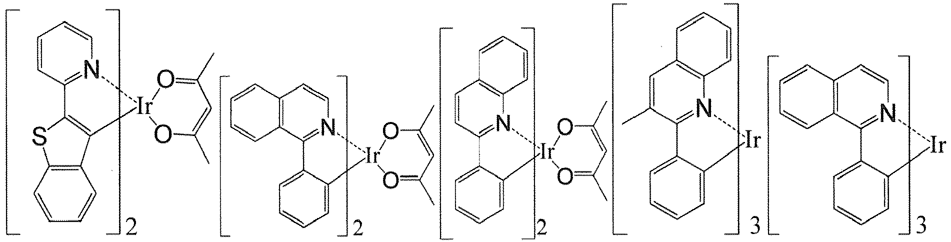

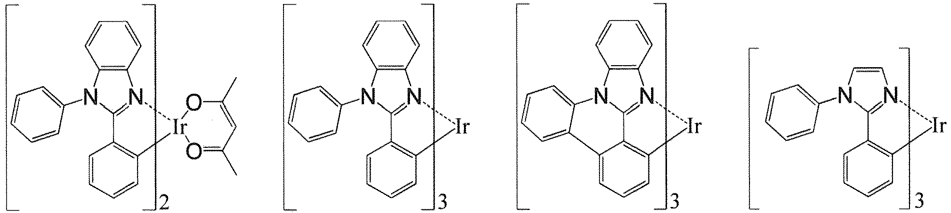

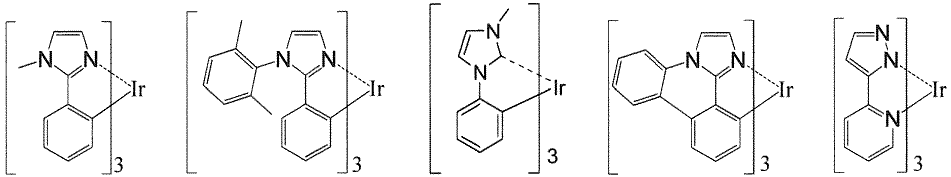

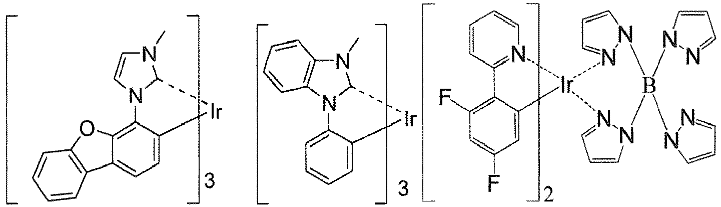

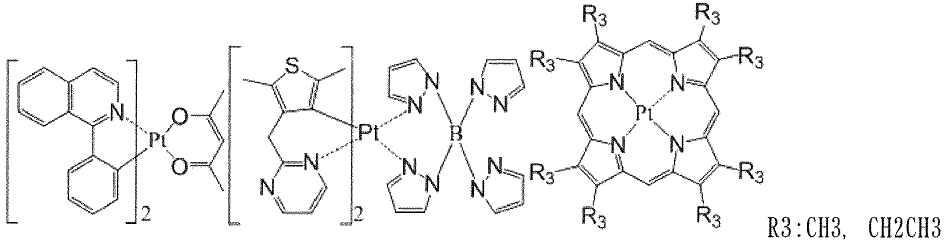

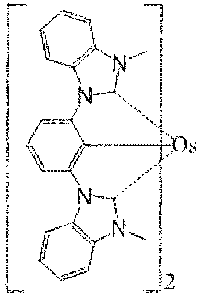

The content of the phosphorescent light-emitting dopant in the light-emitting layer desirably falls within the range of from 2 wt% to 40 wt%, preferably from 5 wt% to 30 wt%.

The thickness of the light-emitting layer, which is not particularly limited, is typically from 1 nm to 300 nm, preferably from 5 nm to 100 nm, and a thin film serving as the layer is formed by the same method as that for the hole-transporting layer.

### (7) Electron-transporting Layer

The electron-transporting layer 6 is formed between the light-emitting layer 5 and the cathode 8 for the purpose of additionally improving the luminous efficiency of the device. A material for the electron-transporting layer is preferably an electron-transportable material that enables smooth injection of an electron from the cathode, and an arbitrary material that has been generally used can be used. Examples of the electron-transporting material that satisfies such condition include a metal complex such as Alq3, a metal complex of 10-hydroxybenzo[h]quinoline, an oxadiazole derivative, a distyrylbiphenyl derivative, a silole derivative, a 3- or 5-hydroxyflavone metal complex, a benzoxazole metal complex, a benzothiazole metal complex, trisbenzimidazolylbenzene, a quinoxaline compound, a phenanthroline derivative, 2-t-butyl-9,10-N,N'-dicyanoanthraquinonediimine, n-type hydrogenated amorphous silicon carbide, n-type zinc sulfide, and n-type zinc selenide.

The thickness of the electron-transporting layer is typically from 1 nm to 300 nm, preferably from 5 nm to 100 nm. The electron-transporting layer is formed through lamination on the light-emitting layer by the application method or the vacuum deposition method as in the hole-transporting layer. The vacuum deposition method is typically employed.

### (8) Cathode

The cathode 8 serves to inject an electron into the electron-transporting layer 6. Although the material to be used in the anode 2 can be used as a material to be used as the cathode, a metal having a low work function is preferred for efficient electron injection, and a proper metal, suchastin, magnesium, indium, calcium, aluminum, or silver, or an alloy thereof is used. Specific examples of the cathode include low-work function alloy electrodes made of a magnesium-silver alloy, a magnesium-indium alloy, and an aluminum-lithium alloy.

The thickness of the cathode is typically the same as that of the anode. When a metal layer that has a high work function and is stable against the air is further laminated on the cathode formed of a low-work function metal for the purpose of protecting the cathode, the stability of the device is improved. To this end, a metal such as aluminum, silver, copper, nickel, chromium, gold, or platinum is used.

Further insertion of an extremely thin insulating film (having a thickness of from 0.1 nm to 5 nm) made of LiF, MgF₂, Li₂O, or the like as the electron-injecting layer 7 between the cathode 8 and the electron-transporting layer 6 is also an effective method of improving the efficiency of the device.

A structure in inverse relation to that illustrated in FIG. 1 is permitted, i.e., the cathode 8, the electron-injecting layer 7, the electron-transporting layer 6, the light-emitting layer 5, the hole-transporting layer 4, the hole-injecting layer 3, and the anode 2 may be laminated in the stated order on the substrate 1, and as described above, an organic EL device may be arranged between two substrates, at least one of which has high transparency. In this case as well, a layer may be added or omitted as required.

The organic EL device of the present invention can be any one of a single device, a device formed of structures arranged in an array manner, and a structure in which the anode and the cathode are arranged in an X-Y matrix manner. According to the organic EL device of the present invention, through the use of a mixed host including specific two kinds of compounds in the light-emitting layer, a device that has high luminous efficiency and is significantly improved in driving stability while being driven at a low voltage is obtained, and the device can exhibit excellent performance in its application to a full-color or multi-color panel.

The present invention is described in more detail below by way of Examples. However, the present invention is not limited to Examples below, and can be carried out in various modes as long as the modes do not deviate from the gist thereof.

### Examples

### Example 1

Each thin film was laminated by a vacuum deposition method at a degree of vacuum of 4.0×10⁻⁴ Pa on a glass substrate on which an anode formed of ITO having a thickness of 150 nm had been formed. First, copper phthalocyanine (CuPc) was formed into a hole-injecting layer having a thickness of 20 nm on the ITO. Next, 4,4-bis[N-(1-naphthyl)-N-phenylamino]biphenyl (NPB) was formed into a hole-transporting layer having a thickness of 20 nm. Next, Compound 1-21 serving as a first host (H1), Compound 3-14 serving as a second host (H2), and tris(2-phenylpyridine)iridium(III) (Ir (PPy) 3) serving as a light-emitting layer guest were co-deposited from vapor deposition sources different from each other to form a light-emitting layer having a thickness of 30 nm. At this time, a vapor deposition rate ratio (volume rate ratio among vaporized products) among the first host, the second host, and Ir(PPy)₃ was 47:47:6. Next, aluminum(III) bis(2-methyl-8-quinolinato)-4-phenylphenolate (BAlq) was formed into a hole-blocking layer having a thickness of 10 nm. Next, tris- (8-hydroxyquinolinato) aluminum (III) (Alq₃) was formed into an electron-transporting layer having a thickness of 40 nm. Further, lithium fluoride (LiF) was formed into an electron-injecting layer having a thickness of 0.5 nm on the electron-transporting layer. Finally, aluminum (Al) was formed into a cathode having a thickness of 100 nm on the electron-inj ecting layer. Thus, an organic EL device was produced.

An external power source was connected to the resultant organic EL device to apply a DC voltage thereto. As a result, an emission spectrum having a local maximum wavelength of 517 nm was observed, and hence it was found that light emission from Ir (PPy) ₃ was obtained. The luminance, current efficiency, power efficiency, and 10% luminance reduction lifetime of the produced organic EL device are shown in Table 1.

The EA of each compound shown in Table 1 was determined from a difference between an ionization potential (IP) measured by photoelectron spectroscopy (manufactured by Riken Keiki Co., Ltd. , AC-2) and an energy gap estimated from an absorption edge of a UV absorption spectrum. The same holds true for Tables 2, 3, and 6 unless otherwise stated.

### Examples 2 to 6 (Note that examples 4-6 are not forming part of the claimed invention)

Organic EL devices were each produced in the same manner as in Example 1 except that in Example 1, a compound shown in Table 1 was used as the second host for the light-emitting layer. An external power source was connected to each of the resultant organic EL devices to apply a DC voltage thereto. As a result, an emission spectrum having a local maximum wavelength of 517 nm from each of the organic EL devices was observed, and hence it was found that light emission from Ir (PPy) ₃ was obtained. The luminance, current efficiency, power efficiency, and 10% luminance reduction lifetime of each of the produced organic EL devices are shown in Table 1.

### Comparative Examples 1 to 4

Organic EL devices were each produced in the same manner as in Example 1 except that in Example 1, a compound shown in Table 1 was used alone as the light-emitting layer host. A host amount was set to the same amount as the total of the first host and second host in Example 1, and a guest amount was similarly set. A power source was connected to each of the resultant organic EL devices to apply a DC voltage thereto. As a result, an emission spectrum having a local maximum wavelength of 517 nm from each of the organic EL devices was observed, and hence it was found that light emission from Ir(PPy)₃ was obtained.

### Comparative Example 5

Each thin film was laminated by a vacuum deposition method at a degree of vacuum of 4.0×10⁻⁴ Pa on a glass substrate on which an anode formed of ITO having a thickness of 150 nm had been formed. First, CuPc was formed into a hole-injecting layer having a thickness of 20 nm on the ITO. Next, NPB was formed into a hole-transporting layer having a thickness of 20 nm. Next, Compound 1-21 serving as a first host, Compound A shown below serving as a second host, and Ir(PPy)₃ serving as a light-emitting layer guest were co-deposited from vapor deposition sources different from each other to form a light-emitting layer having a thickness of 30 nm. At this time, a vapor deposition rate ratio among the first host, the second host, and Ir (PPy) ₃ was 47:47: 6. Next, BAlq was formed into a hole-blocking layer having a thickness of 10 nm. Next, Alq₃ was formed into an electron-transporting layer having a thickness of 40 nm. Further, lithium fluoride (LiF) was formed into an electron-injecting layer having a thickness of 0.5 nm on the electron-transporting layer. Finally, aluminum (Al) was formed into a cathode having a thickness of 100 nm on the electron-injecting layer. Thus, an organic EL device was produced.

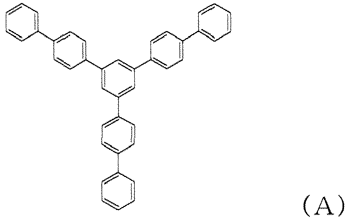

### Comparative Example 6

An organic EL device using Compound A alone as a light-emitting layer host was produced in the same manner as in Comparative Example 5.

An external power source was connected to the resultant organic EL device to apply a DC voltage thereto. As a result, an emission spectrum having a local maximum wavelength of 517 nm from the organic EL device was observed, and hence it was found that light emission from Ir(PPy)₃ was obtained. The luminance, current efficiency, power efficiency, and 10% luminance reduction lifetime of the produced organic EL device are shown in Table 2.

The luminance, current efficiency, power efficiency, and 10% luminance reduction lifetime of the produced organic EL device are shown in Table 1. The luminance, the current efficiency, and the power efficiency are values at a driving current of 20 mA/cm², and are initial characteristics. The 10% luminance reduction time is a value obtained by converting a value at a driving current of 40 mA/cm² into a value at an initial luminance of 9,000 cd/m² with an acceleration factor of 1.8, and is a lifetime characteristic. Compound Nos. used for the first host (H1) and the second host (H2) are numbers attached to the above-mentioned chemical formulae. The same holds true for Tables 2 to 6 unless otherwise stated.

**Table 1 (Note that examples 4-6 are not forming part of the claimed invention)**

| | H1 Compoun d No. | H2 Compoun d No. | Luminance (cd/m²) | Current efficiency (cd/A) | Power efficiency (lm/W) | 10% luminance reduction time (h) |
|---|---|---|---|---|---|---|
| Ex. 1 | 1-21 | 3-14 | 10,972 | 54.9 | 38.3 | 388 |
| Ex. 2 | 1-21 | 3-2 | 10,655 | 53.3 | 35.6 | 349 |
| Ex. 3 | 1-21 | 3-24 | 11,132 | 55.7 | 38.0 | 413 |
| Ex. 4 | 1-21 | 3-51 | 12,069 | 56.3 | 43.2 | 414 |
| Ex. 5 | 1-21 | 3-52 | 9,590 | 56.8 | 34.1 | 389 |
| Ex. 6 | 1-21 | 3-54 | 10,130 | 56.0 | 34.7 | 401 |
| Comp. Ex. 1 | 1-21 | | 10,335 | 51.7 | 38.7 | 259 |
| Comp. Ex. 2 | 3-14 | | 7,296 | 36.5 | 19.1 | 127 |
| Comp. Ex.3 | 3-2 | | 7,002 | 35.0 | 18.0 | 90 |
| Comp. Ex.4 | 3-24 | | 7,149 | 35.7 | 18.4 | 153 |
| Comp. Ex.5 | 1-21 | A | 10,240 | 51.2 | 32.8 | 232 |
| Comp. Ex.6 | A | | 6,782 | 33.9 | 17.2 | 264 |

As can be seen from comparison between Examples 1 to 3 and Comparative Examples 1 to 4 in Table 1, the use of two kinds of compounds each having a specific skeleton as light-emitting layer hosts significantly improves the luminance, the current efficiency, the power efficiency, and the 10% luminance reduction lifetime. Those results have revealed that according to the present invention, an organic EL phosphorescent device showing high efficiency and a satisfactory lifetime characteristic can be achieved.

### Example 7

Each thin film was laminated by a vacuum deposition method at a degree of vacuum of 4.0×10⁻⁴ Pa on a glass substrate on which an anode formed of ITO having a thickness of 150 nm had been formed. First, CuPc was formed into a hole-injecting layer having a thickness of 20 nm on the ITO. Next, NPB was formed into a hole-transporting layer having a thickness of 20 nm. Next, Compound 1-18 serving as a first host, Compound 3-14 serving as a second host, and Ir (PPy) ₃ serving as a light-emitting layer guest were co-deposited from vapor deposition sources different from each other to form a light-emitting layer having a thickness of 30 nm. At this time, a vapor deposition rate ratio among the first host, the second host, and Ir (PPy) ₃ was 47:47:6. Next, BAlq was formed into a hole-blocking layer having a thickness of 10 nm. Next, Alq₃ was formed into an electron-transporting layer having a thickness of 40 nm. Further, lithium fluoride (LiF) was formed into an electron-injecting layer having a thickness of 0.5 nm on the electron-transporting layer. Finally, aluminum (Al) was formed into a cathode having a thickness of 100 nm on the electron-injecting layer. Thus, an organic EL device was produced.

An external power source was connected to the resultant organic EL device to apply a DC voltage thereto. As a result, an emission spectrum having a local maximum wavelength of 517 nm was observed, and hence it was found that light emission from Ir (PPy) ₃ was obtained.

### Examples 8 to 12 (Note that examples 10-12 are not forming part of the claimed invention)

Organic EL devices were each produced in the same manner as in Example 7 except that a compound shown in Table 2 was used as the second host for the light-emitting layer. An external power source was connected to each of the resultant organic EL devices to apply a DC voltage thereto. As a result, an emission spectrum having a local maximum wavelength of 517 nm from each of the organic EL devices was observed, and hence it was found that light emission from Ir(PPy)₃ was obtained.

### Comparative Examples 7 to 10

Organic EL devices were each produced in the same manner as in Example 7 except that a compound shown in Table 2 was used alone as the light-emitting layer host. A host amount was set to the same amount as the total of the first host and second host in Example 4. An external power source was connected to each of the resultant organic EL devices to apply a DC voltage thereto. As a result, an emission spectrum having a local maximum wavelength of 517 nm from each of the organic EL devices was observed, and hence it was found that light emission from Ir(PPy)₃ was obtained.

The luminance, current efficiency, power efficiency, and 10% luminance reduction lifetime of each of the produced organic EL devices are shown in Table 2.

**Table 2 (Note that examples 10-12 are not forming part of the claimed invention)**

| | H1 Compound No. | H2 Compound No. | Luminance (cd/m²) | Current efficiency (cd/A) | Power efficiency (lm/W) | 10% luminance reduction time (h) |
|---|---|---|---|---|---|---|
| Ex. 7 | 1-18 | 3-14 | 10,295 | 51.5 | 39.4 | 434 |
| Ex. 8 | 1-18 | 3-2 | 10,335 | 51.7 | 37.8 | 420 |
| Ex. 9 | 1-18 | 3-24 | 10,922 | 54.6 | 40.8 | 467 |
| Ex. 10 | 1-18 | 3-51 | 11,325 | 52.8 | 44.5 | 464 |
| Ex. 11 | 1-18 | 3-52 | 9,302 | 55.1 | 36.2 | 468 |
| Ex. 12 | 1-18 | 3-54 | 9,939 | 54.9 | 37.3 | 454 |
| Comp. Ex. 7 | 1-18 | | 11,089 | 55.4 | 44.7 | 282 |
| Comp. Ex.8 | 3-14 | | 6,846 | 34.2 | 17.9 | 173 |
| Comp. Ex.9 | 3-2 | | 6,570 | 32.9 | 16.9 | 162 |
| Comp. Ex.10 | 3-24 | | 6,708 | 33.5 | 17.3 | 207 |

As can be seen from comparison between Examples 7 to 9 and Comparative Examples 7 to 10 in Table 2, the use of two kinds of compounds each having a specific skeleton as light-emitting layer hosts significantly improves the luminance, the current efficiency, the power efficiency, and the 10% luminance reduction lifetime.

### Example 13

Each thin film was laminated by a vacuum deposition method at a degree of vacuum of 4.0×10⁻⁴ Pa on a glass substrate on which an anode formed of ITO having a thickness of 150 nm had been formed. First, CuPc was formed into a hole-injecting layer having a thickness of 20 nm on the ITO. Next, NPB was formed into a hole-transporting layer having a thickness of 20 nm. Next, Compound 2-5 serving as a first host, Compound 3-14 serving as a second host, and Ir(PPy)₃ serving as a light-emitting layer guest were co-deposited from vapor deposition sources different from each other to form a light-emitting layer having a thickness of 30 nm. At this time, a vapor deposition rate ratio among the first host, the second host, and Ir (PPy) ₃ was 47:47:6. Next, BAlq was formed into a hole-blocking layer having a thickness of 10 nm. Next, Alq₃ was formed into an electron-transporting layer having a thickness of 40 nm. Further, lithium fluoride (LiF) was formed into an electron-injecting layer having a thickness of 0.5 nm on the electron-transporting layer. Finally, aluminum (Al) was formed into a cathode having a thickness of 100 nm on the electron-inj ecting layer. Thus, an organic EL device was produced.

An external power source was connected to the resultant organic EL device to apply a DC voltage thereto. As a result, an emission spectrum having a local maximum wavelength of 517 nm was observed, and hence it was found that light emission from Ir (PPy) ₃ was obtained.

### Examples 14 to 18 (Note that examples 16-18 are not forming part of the claimed invention)

Organic EL devices were each produced in the same manner as in Example 13 except that a compound shown in Table 3 was used as the second host for the light-emitting layer. An external power source was connected to each of the resultant organic EL devices to apply a DC voltage thereto. As a result, an emission spectrum having a local maximum wavelength of 517 nm from each of the organic EL devices was observed, and hence it was found that light emission from Ir(PPy)₃ was obtained.

### Comparative Examples 11 to 14

Organic EL devices were each produced in the same manner as in Example 13 except that a compound shown in Table 3 was used alone as the light-emitting layer host. A host amount was set to the same amount as the total of the first host and the second host in Example 7. An external power source was connected to each of the resultant organic EL devices to apply a DC voltage thereto. As a result, an emission spectrum having a local maximum wavelength of 517 nm from each of the organic EL devices was observed, and hence it was found that light emission from Ir(PPy)₃ was obtained.

The luminance, current efficiency, power efficiency, and 10% luminance reduction lifetime of each of the produced organic EL devices are shown in Table 3.

**Table 3 (Note that examples 16-18 are not forming part of the claimed invention)**

| | H1 Compound No. | H2 Compound No. | Luminance (cd/m²) | Current efficiency (cd/A) | Power efficiency (lm/W) | 10% luminance reduction time (h) |
|---|---|---|---|---|---|---|
| Ex. 13 | 2-5 | 3-14 | 9,817 | 49.1 | 35.9 | 298 |
| Ex. 14 | 2-5 | 3-2 | 9,782 | 48.9 | 34.1 | 280 |
| Ex. 15 | 2-5 | 3-24 | 10,986 | 54.9 | 39.2 | 312 |
| Ex. 16 | 2-5 | 3-51 | 10,799 | 50.4 | 40.5 | 320 |
| Ex. 17 | 2-5 | 3-52 | 8,804 | 52.1 | 32.7 | 300 |
| Ex. 18 | 2-5 | 3-54 | 9,997 | 55.2 | 35.8 | 309 |
| Comp. Ex. 11 | 2-5 | | 9,860 | 49.3 | 38.7 | 171 |
| Comp. Ex. 12 | 3-14 | | 7,296 | 36.5 | 19.1 | 127 |
| Comp. Ex. 13 | 3-2 | | 7,002 | 35.0 | 18.0 | 90 |
| Comp. Ex. 14 | 3-24 | | 7,149 | 35.7 | 18.4 | 153 |

As can be seen from Table 3, Examples 13 to 15 are each significantly excellent in luminance, current efficiency, power efficiency, and 10% luminance reduction lifetime.

### Reference Example 1

Compound 1-21 was vapor-deposited on a glass substrate by a vacuum deposition method at a degree of vacuum of 4.0×10⁻⁴ Pa and a vapor deposition rate of 2 Å/sec. As a result, its temperature (vaporization temperature) at this time was 303°C.

Results obtained by similarly measuring the vaporization temperatures of compounds shown in Table 4 are shown in Table 4.

**Table 4**

| Compound No. | Vaporization temperature (°C) |
|---|---|
| 1-21 | 303 |
| 1-18 | 284 |
| 3-14 | 294 |
| 3-24 | 301 |
| 3-2 | 246 |
| 3-51 | 295 |
| 3-52 | 284 |
| 3-54 | 298 |

### Reference Example 21

Compound 1-21 serving as the first host (H1) and Compound 3-14 serving as the second host (H2) were preliminarily mixed at a weight ratio of 1:1. The mixture was dissolved in tetrahydrofuran and separated by liquid chromatography, followed by the detection of the peaks of the respective components with a UV-visible spectrophotometric detector. The mixing ratios (peak area ratios) of Compounds 1-21 and 3-14 were determined from values for the areas of the detected peaks. As a result, the mixing ratios were 48.2% and 51.8%, respectively (mixing ratios before vapor deposition).

The mixture was formed into a film having a thickness of 500 nm from one vapor deposition source on a glass substrate by a vacuum deposition method at a degree of vacuum of 4.0×10⁻⁴ Pa and a vapor deposition rate of 2 Å/sec. The resultant deposited film was extracted from the glass substrate with tetrahydrofuran, and the mixing ratios (peak area ratios) of Compounds 1-21 and 3-14 were determined by liquid chromatography in the same manner as that described above . As a result, the mixing ratios were 49.3% and 50.7%, respectively (mixing ratios of the deposited film) . A mixing ratio change amount (ΔR) is 1.1%. The mixing ratio change amount is determined from a difference between the % of the first host or the second host before the vapor deposition and the % thereof after the vapor deposition. In the case of the first host, the difference is calculated to be 48.2%-49.3%=-1.1%, and the absolute value of the calculated value is the change amount.

### Reference Examples 22 to 28

Mixing ratio change amounts were each similarly calculated by using compounds shown in Table 5 as the first host and the second host. Mixing ratios before vapor deposition, the mixing ratios of a deposited film, a mixing ratio change amount (ΔR), and a difference (ΔT) in vaporization temperature between the first compound and the second compound calculated from Table 4 are shown in Table 5.

**Table 5**

| No. | H1 Compound | H2 Compound | H1/H2 ratio (before vapor deposition) | H1/H2 ratio (after vapor deposition) | ΔR (%) | ΔT (°C) |
|---|---|---|---|---|---|---|
| 21 | 1-21 | 3-14 | 48.2/51.8 | 49.3/50.7 | 1.1 | 9 |
| 22 | 1-21 | 3-24 | 43.5/56.5 | 44.4/55.6 | 0.9 | 2 |
| 23 | 1-21 | 3-51 | 47.7/52.3 | 46.2/53.8 | 1.5 | 8 |
| 24 | 1-18 | 3-14 | 47.9/52.1 | 45.5/54.5 | 2.4 | -11 |
| 25 | 1-18 | 3-24 | 43.8/56.2 | 41.0/59.0 | 2.8 | -17 |
| 26 | 1-18 | 3-51 | 48.6/51.4 | 46.7/53.3 | 1.9 | -11 |
| 27 | 1-21 | 3-2 | 44.0/56.0 | 51.9/48.1 | 7.9 | 57 |
| 28 | 1-18 | 3-2 | 42.8/57.2 | 48.5/51.5 | 5.7 | 38 |

As is apparent from Table 5, when two kinds of compounds whose difference in vaporization temperature is within 30°C are preliminarily mixed, and are formed into a film from one vapor deposition source, the film can be formed so that a mixing ratio change amount may be within 5%.

### Example 19

Each thin film was laminated by a vacuum deposition method at a degree of vacuum of 4.0×10⁻⁴ Pa on a glass substrate on which an anode formed of ITO having a thickness of 150 nm had been formed. First, CuPC was formed into a hole-inj ecting layer having a thickness of 20 nm on the ITO. Next, NPB was formed into a hole-transporting layer having a thickness of 20 nm. Next, a mixed host obtained by preliminarily mixing Compound 1-21 and Compound 3-14, and Ir (PPy) ₃ serving as a light-emitting layer guest were co-deposited from vapor deposition sources different from each other to form a light-emitting layer having a thickness of 30 nm. At this time, a vapor deposition rate ratio between the mixed host and Ir (PPy) ₃ was 94:6. Next, BAlq was formed into a hole-blocking layer having a thickness of 10 nm. Next, Alq₃ was formed into an electron-transporting layer having a thickness of 40 nm. Further, lithium fluoride (LiF) was formed into an electron-injecting layer having a thickness of 0.5 nm on the electron-transporting layer. Finally, aluminum (Al) was formed into a cathode having a thickness of 100 nm on the electron-injecting layer. Thus, an organic EL device was produced.

An external power source was connected to the resultant organic EL device to apply a DC voltage thereto. As a result, an emission spectrum having a local maximum wavelength of 517 nm was observed, and hence it was found that light emission from Ir (PPy) ₃ was obtained.

### Examples 20 to 22 (Note that examples 21 and 22 are not forming part of the claimed invention)

Organic EL devices were each produced in the same manner as in Example 19 except that compounds shown in Table 6 were used as the mixed host. An external power source was connected to each of the resultant organic EL devices to apply a DC voltage thereto. As a result, an emission spectrum having a local maximum wavelength of 517 nm from each of the organic EL devices was observed, and hence it was found that light emission from Ir (PPy) 3 was obtained.

### Comparative Examples 17 and 19

Organic EL devices were each produced in the same manner as in Example 19 except that a compound shown in Table 6 was used alone as the light-emitting layer host. A host amount was set to the same amount as the mixed host in Example 19. An external power source was connected to each of the resultant organic EL devices to apply a DC voltage thereto. As a result, an emission spectrum having a local maximum wavelength of 517 nm from each of the organic EL devices was observed, and hence it was found that light emission from Ir (PPy) ₃ was obtained.

The luminance, current efficiency, power efficiency, and 10% luminance reduction lifetime of each of the produced organic EL devices are shown in Table 6.

**Table 6 (Note that examples 21 and 22 are not forming part of the claimed invention)**

| | H1 Compound No. | H2 Compound No. | Luminance (cd/m²) | Current efficiency (cd/A) | Power efficiency (lm/W) | 10% luminance reduction time (h) |
|---|---|---|---|---|---|---|
| Ex. 19 | 1-21 | 3-14 | 10,678 | 53.4 | 37.3 | 381 |
| Ex. 20 | 1-21 | 3-24 | 11,100 | 55.5 | 37.9 | 430 |
| Ex. 21 | 1-21 | 3-51 | 9,707 | 52.1 | 42.1 | 407 |
| Ex. 22 | 1-18 | 3-51 | 11,292 | 52.6 | 44.3 | 483 |
| Comp. Ex.17 | 1-21 | | 10,335 | 51.7 | 38.7 | 259 |
| Comp. Ex.18 | 3-14 | | 7,296 | 36.5 | 19.1 | 127 |
| Comp. Ex.19 | 3-24 | | 7,149 | 35.7 | 18.4 | 153 |

As can be seen from Table 6, when two kinds of compounds each having a specific skeleton are preliminarily mixed, and are used as a mixed host, the luminance, the current efficiency, the power efficiency, and the 10% luminance reduction lifetime are significantly improved.

The electron affinities (EA) of the compounds used in Examples and Comparative Examples are shown in Table 7.

**Table 7**

| Compound No. | EA (eV) | Compound No. | EA (eV) |
|---|---|---|---|
| 1-18 | 2.77 | 3-24 | 2.17 |
| 1-21 | 2.80 | 3-51 | 2.25 |
| 2-5 | 2.73 | 3-52 | 2.33 |
| 3-2 | 2.15 | 3-54 | 2.20 |
| 3-14 | 2.19 | A | 2.12 |

### Industrial Applicability

The organic EL device of the present invention suppresses energy outflow from the inside of the light-emitting layer and can achieve high efficiency and a long lifetime, and hence the device has a high technological value in its application to, for example, flat panel displays (such as a cellular phone display device, an on-vehicle display device, an OA computer display device, and a television), light sources each taking advantage of its feature as a surface emitter (such as illumination, a light source for a copying machine, and backlight sources for a liquid crystal display and meters), display boards, and marker lamps.

### Reference Signs List

- 1: substrate
- 2: anode
- 3: hole-injecting layer
- 4: hole-transporting layer
- 5: light-emitting layer
- 6: electron-transporting layer
- 7: electron-injecting layer
- 8: cathode

## Claims

1. An organic electroluminescent device, comprising one or more light-emitting layers [5] between an anode [2] and a cathode [8] opposite to each other, wherein: at least one of the light-emitting layers [5] contains at least two kinds of host materials and at least one kind of light-emitting dopant; and at least one kind of the host materials comprises a host material (H1) selected from compounds each represented by any one of the following general formulae (1) and (2), and at least one kind of the host materials comprises a host material (H2) selected from compounds each represented by the following general formula (3): 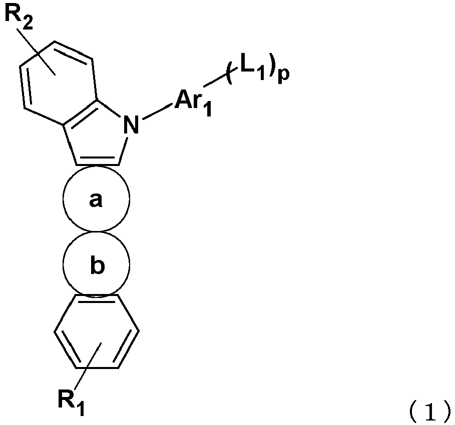 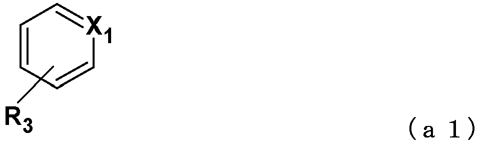 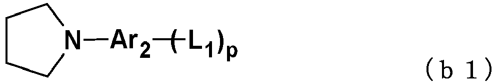 wherein:
a ring a represents an aromatic ring or a heterocycle represented by the formula (a1) that is fused at arbitrary positions of two adjacent rings, X₁ represents C-R or N, a ring b represents a heterocycle represented by the formula (b1) that is fused at arbitrary positions of two adjacent rings, Ar₁ and Ar₂ each independently represent an aromatic hydrocarbon group having 6 to 24 carbon atoms, or an aromatic heterocyclic group having 3 to 16 carbon atoms, L₁ represents an aromatic hydrocarbon group having 6 to 24 carbon atoms, an aromatic heterocyclic group having 3 to 16 carbon atoms, or a linked aromatic group obtained by linking 2 to 10 aromatic rings of the groups, and the aromatic hydrocarbon group, the aromatic heterocyclic group, or the linked aromatic group in Ar₁, Ar₂, and L₁ may have a substituent;
p represents an integer of from 0 to 7, provided that when p represents 2 or more, L₁'s may be identical to or different from each other; and
R and R₁ to R₃ each independently represent hydrogen, an alkyl group having 1 to 20 carbon atoms, an aralkyl group having 7 to 38 carbon atoms, an alkenyl group having 2 to 20 carbon atoms, an alkynyl group having 2 to 20 carbon atoms, a dialkylamino group having 2 to 40 carbon atoms, a diarylamino group having 12 to 44 carbon atoms, a diaralkylamino group having 14 to 76 carbon atoms, an acyl group having 2 to 20 carbon atoms, an acyloxy group having 2 to 20 carbon atoms, an alkoxy group having 1 to 20 carbon atoms, an alkoxycarbonyl group having 2 to 20 carbon atoms, an alkoxycarbonyloxy group having 2 to 20 carbon atoms, an alkylsulfonyl group having 1 to 20 carbon atoms, an aromatic hydrocarbon group having 6 to 24 carbon atoms, or an aromatic heterocyclic group having 3 to 16 carbon atoms, and may each have a substituent; 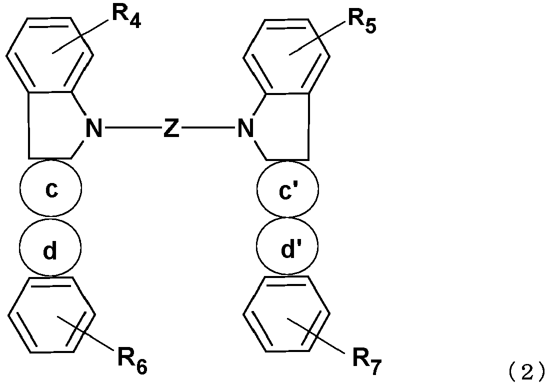 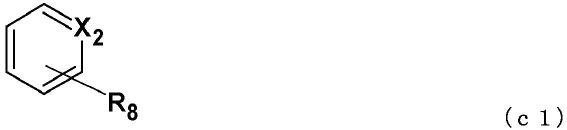 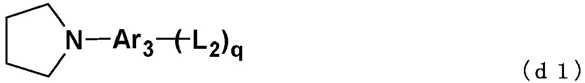
wherein:
a ring c and a ring c' each represent an aromatic ring or a heterocycle represented by the formula (c1) that is fused at arbitrary positions of adjacent rings, a ring d and a ring d' each represent a heterocycle represented by the formula (d1) that is fused at arbitrary positions of adjacent rings, and the ring c and the ring c', or the ring d and the ring d' may be identical to or different from each other;
X₂ represents C-R' or N, Z represents an aromatic hydrocarbon group having 6 to 24 carbon atoms, an aromatic heterocyclic group having 3 to 16 carbon atoms, or a divalent linked aromatic group obtained by linking 2 to 10 aromatic rings of the groups, Ar₃ represents an aromatic hydrocarbon group having 6 to 24 carbon atoms, or a monocyclic aromatic heterocyclic group having 3 to 6 carbon atoms, L₂ represents an aromatic hydrocarbon group having 6 to 24 carbon atoms, an aromatic heterocyclic group having 3 to 18 carbon atoms, or a linked aromatic group obtained by linking 2 to 10 aromatic rings of the groups, and the aromatic hydrocarbon group, the aromatic heterocyclic group, or the linked aromatic group in Z, Ar₃, and L₂ may have a substituent;
q represents an integer of from 0 to 7, provided that when q represents 2 or more, L₂'s may be identical to or different from each other; and
R' and R₄ to R₈ each independently represent hydrogen, an alkyl group having 1 to 20 carbon atoms, an aralkyl group having 7 to 38 carbon atoms, an alkenyl group having 2 to 20 carbon atoms, an alkynyl group having 2 to 20 carbon atoms, a dialkylamino group having 2 to 40 carbon atoms, a diarylamino group having 12 to 44 carbon atoms, a diaralkylamino group having 14 to 76 carbon atoms, an acyl group having 2 to 20 carbon atoms, an acyloxy group having 2 to 20 carbon atoms, an alkoxy group having 1 to 20 carbon atoms, an alkoxycarbonyl group having 2 to 20 carbon atoms, an alkoxycarbonyloxy group having 2 to 20 carbon atoms, an alkylsulfonyl group having 1 to 20 carbon atoms, an aromatic hydrocarbon group having 6 to 24 carbon atoms, or an aromatic heterocyclic group having 3 to 16 carbon atoms, and may each have a substituent; 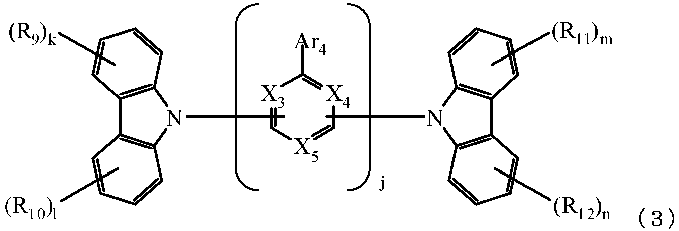 wherein:
R₉ to R₁₂ each independently represent an aromatic hydrocarbon group having 6 to 18 carbon atoms or an aromatic heterocyclic group having 3 to 9 carbon atoms, and may each have a substituent;
Ar₄'s each independently represent hydrogen or an aromatic hydrocarbon group having 6 to 24 carbon atoms, and the aromatic hydrocarbon group may have a substituent, j represents an integer of 1 or 3, and at least one Ar₄ does not represent hydrogen;
X₃ to X₅ each independently represent N, C-R", or C-, and R"'s each independently represent hydrogen, an alkyl group having 1 to 20 carbon atoms, an alkoxy group having 1 to 20 carbon atoms, or a diarylamino group having 12 to 44 carbon atoms; and
k, 1, m, and n represent integers satisfying 0 ≤ k+l+m+n ≤ 16.

2. An organic electroluminescent device according to claim 1, wherein one of the two host materials comprises a host material selected from the compounds each represented by any one of the general formulae (1) and (2), another one of the two host materials comprises a host material selected from the compounds each represented by the general formula (3), and a difference in electron affinity (ΔEA) between the two host materials is more than 0.1 eV.

3. An organic electroluminescent device according to claim 1, wherein in the general formula (1), X₁ represents C-R.

4. An organic electroluminescent device according to claim 1, wherein in the general formula (2), X₂ represents C-R'.

5. An organic electroluminescent device according to claim 1, wherein in the general formula (1), at least one of Ar₁ or Ar₂ represents a substituted or unsubstituted aromatic heterocyclic group having 3 to 9 carbon atoms.

6. An organic electroluminescent device according to claim 1, wherein in the general formula (3), j represents an integer of 1.

7. An organic electroluminescent device according to claim 1, wherein in the general formula (3), X₃ to X₅ each represent C-H, N, or C-.

8. An organic electroluminescent device according to claim 1, wherein a difference in vaporization temperature between the host material (H1) and the host material (H2) is within 30°C.

9. An organic electroluminescent device according to claim 8, wherein the difference in vaporization temperature is within 10°C.

10. An organic electroluminescent device according to any one of claims 1 to 9, wherein the light-emitting dopant comprises a phosphorescent light-emitting dopant comprising an organometallic complex containing at least one metal selected from ruthenium, rhodium, palladium, silver, rhenium, osmium, iridium, platinum, and gold.

11. A method for preparing an organic electroluminescent device according to claim 1, wherein the organic electroluminescent device comprises a light-emitting layer [5], wherein the light-emitting layer [5] is produced by preliminarily mixing the host material (H1) and the host material (H2), and vapor-depositing the mixture from one vapor deposition source.

## Patentansprüche

1. Eine organische Elektrolumineszenzvorrichtung, umfassend eine oder mehrere lichtemittierende Schichten [5] zwischen einer Anode [2] und einer Kathode [8], die einander gegenüberliegen, wobei: mindestens eine der lichtemittierenden Schichten [5] mindestens zwei Arten von Wirtsmaterialien und mindestens eine Art von lichtemittierendem Dotierungsmittel enthält; und mindestens eine Art der Wirtsmaterialien ein Wirtsmaterial (H1), ausgewählt aus Verbindungen, die jeweils durch eine der folgenden allgemeinen Formeln (1) und (2) dargestellt sind, umfasst, und mindestens eine Art der Wirtsmaterialien ein Wirtsmaterial (H2), ausgewählt aus Verbindungen, die jeweils durch die folgende allgemeine Formel (3) dargestellt sind, umfasst: 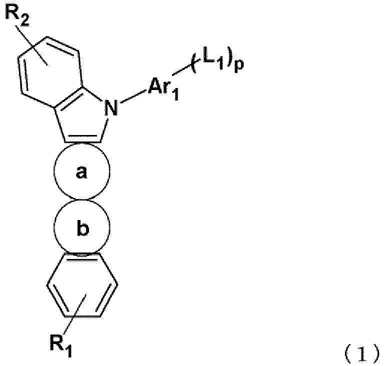 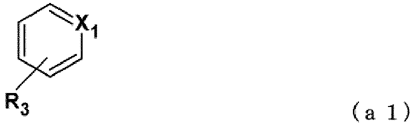 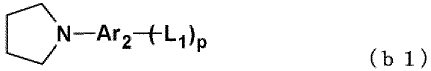 wobei:
ein Ring a einen aromatischen Ring oder einen Heterocyclus, dargestellt durch die Formel (a1), darstellt, der an beliebigen Positionen zweier benachbarter Ringe kondensiert ist, X₁ für C-R oder N steht, ein Ring b einen Heterocyclus, dargestellt durch die Formel (b1), darstellt, der an beliebigen Positionen zweier benachbarter Ringe kondensiert ist, Ar₁ und Ar₂ jeweils unabhängig eine aromatische Kohlenwasserstoffgruppe mit 6 bis 24 Kohlenstoffatomen oder eine aromatische heterocyclische Gruppe mit 3 bis 16 Kohlenstoffatomen darstellen, L₁ eine aromatische Kohlenwasserstoffgruppe mit 6 bis 24 Kohlenstoffatomen, eine aromatische heterocyclische Gruppe mit 3 bis 16 Kohlenstoffatomen oder eine verknüpfte aromatische Gruppe, erhalten durch Verknüpfen von 2 bis 10 aromatischen Ringen der Gruppen, darstellt und die aromatische Kohlenwasserstoffgruppe, die aromatische heterocyclische Gruppe oder die verknüpfte aromatische Gruppe in Ar₁, Ar₂ und L₁ einen Substituenten aufweisen können;
p eine ganze Zahl von 0 bis 7 darstellt, mit der Maßgabe, dass, wenn p 2 oder mehr darstellt, die Reste L₁ identisch oder voneinander verschieden sein können; und
R und R₁ bis R₃ jeweils unabhängig Wasserstoff, eine Alkylgruppe mit 1 bis 20 Kohlenstoffatomen, eine Aralkylgruppe mit 7 bis 38 Kohlenstoffatomen, eine Alkenylgruppe mit 2 bis 20 Kohlenstoffatomen, eine Alkinylgruppe mit 2 bis 20 Kohlenstoffatomen, eine Dialkylaminogruppe mit 2 bis 40 Kohlenstoffatomen, eine Diarylaminogruppe mit 12 bis 44 Kohlenstoffatomen, eine Diaralkylaminogruppe mit 14 bis 76 Kohlenstoffatomen, eine Acylgruppe mit 2 bis 20 Kohlenstoffatomen, eine Acyloxygruppe mit 2 bis 20 Kohlenstoffatomen, eine Alkoxygruppe mit 1 bis 20 Kohlenstoffatomen, eine Alkoxycarbonylgruppe mit 2 bis 20 Kohlenstoffatomen, eine Alkoxycarbonyloxygruppe mit 2 bis 20 Kohlenstoffatomen, eine Alkylsulfonylgruppe mit 1 bis 20 Kohlenstoffatomen, eine aromatische Kohlenwasserstoffgruppe mit 6 bis 24 Kohlenstoffatomen oder eine aromatische heterocyclische Gruppe mit 3 bis 16 Kohlenstoffatomen darstellen und jeweils einen Substituenten aufweisen können; 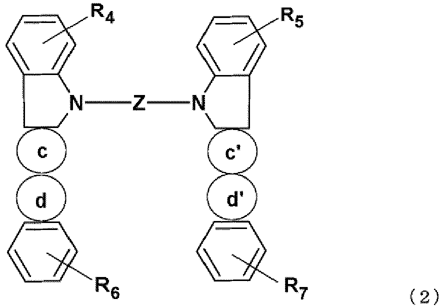 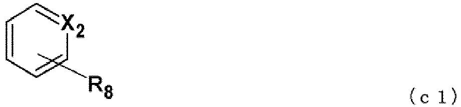 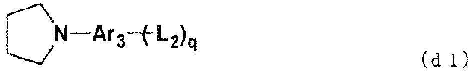 wobei:
ein Ring c und ein Ring c' jeweils einen aromatischen Ring oder einen Heterocyclus, dargestellt durch die Formel (c1), darstellen, der an beliebigen Positionen benachbarter Ringe kondensiert ist, ein Ring d und ein Ring d' jeweils einen Heterocyclus, dargestellt durch die Formel (d1), darstellen, der an beliebigen Positionen benachbarter Ringe kondensiert ist, und der Ring c und der Ring c' oder der Ring d und der Ring d' identisch oder voneinander verschieden sein können;
X₂ für C-R' oder N steht, Z eine aromatische Kohlenwasserstoffgruppe mit 6 bis 24 Kohlenstoffatomen, eine aromatische heterocyclische Gruppe mit 3 bis 16 Kohlenstoffatomen oder eine zweiwertige verknüpfte aromatische Gruppe, erhalten durch Verknüpfen von 2 bis 10 aromatischen Ringen der Gruppen, steht, Ar₃ eine aromatische Kohlenwasserstoffgruppe mit 6 bis 24 Kohlenstoffatomen oder eine monocyclische aromatische heterocyclische Gruppe mit 3 bis 6 Kohlenstoffatomen darstellt, L₂ eine aromatische Kohlenwasserstoffgruppe mit 6 bis 24 Kohlenstoffatomen, eine aromatische heterocyclische Gruppe mit 3 bis 18 Kohlenstoffatomen oder eine verknüpfte aromatische Gruppe, erhalten durch Verknüpfen von 2 bis 10 aromatischen Ringen der Gruppen, darstellt und die aromatische Kohlenwasserstoffgruppe, die aromatische heterocyclische Gruppe oder die verknüpfte aromatische Gruppe in Z, Ar₃ und L₂ einen Substituenten aufweisen können;
q eine ganze Zahl von 0 bis 7 darstellt, mit der Maßgabe, dass, wenn q 2 oder mehr darstellt, die Reste L₂ identisch oder voneinander verschieden sein können; und
R' und R₄ bis R₈ jeweils unabhängig Wasserstoff, eine Alkylgruppe mit 1 bis 20 Kohlenstoffatomen, eine Aralkylgruppe mit 7 bis 38 Kohlenstoffatomen, eine Alkenylgruppe mit 2 bis 20 Kohlenstoffatomen, eine Alkinylgruppe mit 2 bis 20 Kohlenstoffatomen, eine Dialkylaminogruppe mit 2 bis 40 Kohlenstoffatomen, eine Diarylaminogruppe mit 12 bis 44 Kohlenstoffatomen, eine Diaralkylaminogruppe mit 14 bis 76 Kohlenstoffatomen, eine Acylgruppe mit 2 bis 20 Kohlenstoffatomen, eine Acyloxygruppe mit 2 bis 20 Kohlenstoffatomen, eine Alkoxygruppe mit 1 bis 20 Kohlenstoffatomen, eine Alkoxycarbonylgruppe mit 2 bis 20 Kohlenstoffatomen, eine Alkoxycarbonyloxygruppe mit 2 bis 20 Kohlenstoffatomen, eine Alkylsulfonylgruppe mit 1 bis 20 Kohlenstoffatomen, eine aromatische Kohlenwasserstoffgruppe mit 6 bis 24 Kohlenstoffatomen oder eine aromatische heterocyclische Gruppe mit 3 bis 16 Kohlenstoffatomen darstellen und jeweils einen Substituenten aufweisen können; 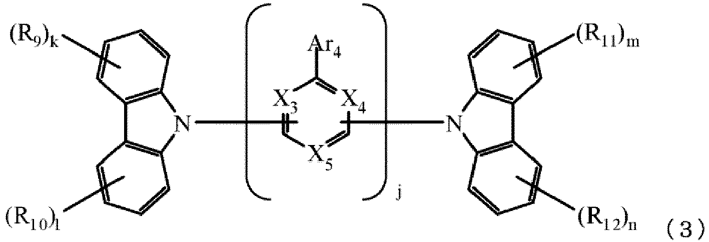
wobei:
Rg bis R₁₂ jeweils unabhängig eine aromatische Kohlenwasserstoffgruppe mit 6 bis 18 Kohlenstoffatomen oder eine aromatische heterocyclische Gruppe mit 3 bis 9 Kohlenstoffatomen darstellen und jeweils einen Substituenten aufweisen können;
die Reste Ar₄ jeweils unabhängig Wasserstoff oder eine aromatische Kohlenwasserstoffgruppe mit 6 bis 24 Kohlenstoffatomen darstellen und die aromatische Kohlenwasserstoffgruppe einen Substituenten aufweisen kann, j eine ganze Zahl von 1 oder 3 darstellt und mindestens ein Ar₄ keinen Wasserstoff darstellt;
X₃ bis X₅ jeweils unabhängig N, C-R" oder C- darstellen und die Reste R" jeweils unabhängig Wasserstoff, eine Alkylgruppe mit 1 bis 20 Kohlenstoffatomen, eine Alkoxygruppe mit 1 bis 20 Kohlenstoffatomen oder eine Diarylaminogruppe mit 12 bis 44 Kohlenstoffatomen darstellen; und
k, l, m und n ganze Zahlen darstellen, die 0 ≤ k+l+m+n ≤ 16 erfüllen.

2. Eine organische Elektrolumineszenzvorrichtung nach Anspruch 1, wobei eines der beiden Wirtsmaterialien ein Wirtsmaterial, ausgewählt aus den Verbindungen, die jeweils durch eine der allgemeinen Formeln (1) und (2) dargestellt sind, umfasst, ein anderes der beiden Wirtsmaterialien ein Wirtsmaterial, ausgewählt aus den Verbindungen, die jeweils durch die allgemeine Formel (3) dargestellt sind, umfasst und eine Differenz der Elektronenaffinität (ΔEA) zwischen den beiden Wirtsmaterialien mehr als 0,1 eV beträgt.

3. Eine organische Elektrolumineszenzvorrichtung nach Anspruch 1, wobei in der allgemeinen Formel (1) X₁ für C-R steht.

4. Eine organische Elektrolumineszenzvorrichtung nach Anspruch 1, wobei in der allgemeinen Formel (2) X₂ für C-R' steht.

5. Eine organische Elektrolumineszenzvorrichtung nach Anspruch 1, wobei in der allgemeinen Formel (1) mindestens eines von Ar₁ oder Ar₂ eine substituierte oder unsubstituierte aromatische heterocyclische Gruppe mit 3 bis 9 Kohlenstoffatomen darstellt.

6. Eine organische Elektrolumineszenzvorrichtung nach Anspruch 1, wobei in der allgemeinen Formel (3) j eine ganze Zahl von 1 darstellt.

7. Eine organische Elektrolumineszenzvorrichtung nach Anspruch 1, wobei in der allgemeinen Formel (3) X₃ bis X₅ jeweils C-H, N oder C- darstellen.

8. Eine organische Elektrolumineszenzvorrichtung nach Anspruch 1, wobei eine Differenz der Verdampfungstemperatur zwischen dem Wirtsmaterial (H1) und dem Wirtsmaterial (H2) innerhalb 30°C liegt.

9. Eine organische Elektrolumineszenzvorrichtung nach Anspruch 8, wobei die Differenz der Verdampfungstemperatur innerhalb 10°C liegt.

10. Eine organische Elektrolumineszenzvorrichtung nach einem der Ansprüche 1 bis 9, wobei das lichtemittierende Dotierungsmittel ein phosphoreszierendes lichtemittierendes Dotierungsmittel umfasst, das einen metallorganischen Komplex umfasst, welcher mindestens ein Metall, ausgewählt aus Ruthenium, Rhodium, Palladium, Silber, Rhenium, Osmium, Iridium, Platin und Gold, enthält.

11. Ein Verfahren zur Herstellung einer organischen Elektrolumineszenzvorrichtung nach Anspruch 1, wobei die organische Elektrolumineszenzvorrichtung eine lichtemittierende Schicht [5] umfasst, wobei die lichtemittierende Schicht [5] durch vorläufiges Mischen des Wirtsmaterials (H1) und des Wirtsmaterials (H2) und Dampfabscheiden des Gemischs aus einer Dampfabscheidequelle hergestellt wird.

## Revendications

1. Dispositif électroluminescent organique, comprenant une ou plusieurs couches luminescentes [5] entre une anode [2] et une cathode [8] opposées l'une à l'autre, dans lequel : au moins une des couches luminescentes [5] contient au moins deux types de matériaux hôtes et au moins un type de dopant luminescent ; et au moins un type des matériaux hôtes comprend un matériau hôte (H1) choisi parmi les composés représentés chacun par l'une quelconque des formules générales (1) et (2) qui suivent, et au moins un type des matériaux hôtes comprend un matériau hôte (H2) choisi parmi les composés représentés chacun par la formule générale (3) qui suit : 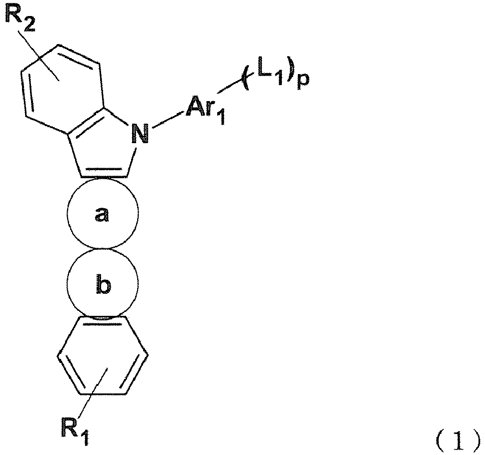 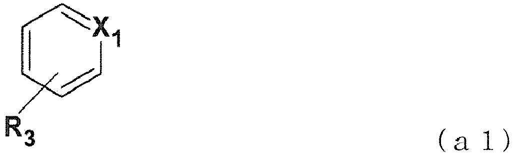 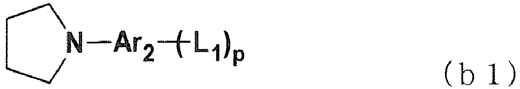 formules dans lesquelles :
le cycle a représente un cycle aromatique ou un hétérocycle représenté par la formule (a1) qui est fusionné au niveau de positions arbitraires de deux cycles adjacents, X₁ représente C-R ou N, le cycle b représente un hétérocycle représenté par la formule (b1) qui est fusionné au niveau de positions arbitraires de deux cycles adjacents, chacun de Ar₁ et Ar₂ représente indépendamment un groupe hydrocarboné aromatique ayant 6 à 24 atomes de carbone, ou un groupe hétérocyclique aromatique ayant 3 à 16 atomes de carbone, L₁ représente un groupe hydrocarboné aromatique ayant 6 à 24 atomes de carbone, un groupe hétérocyclique aromatique ayant 3 à 16 atomes de carbone, ou un groupe aromatique lié obtenu par liaison de 2 à 10 cycles aromatiques des groupes, et le groupe hydrocarboné aromatique, le groupe hétérocyclique aromatique, ou le groupe aromatique lié dans Ar₁, Ar₂ et L₁ peut porter un substituant ;
p représente un entier de 0 à 7, sous réserve que, lorsque p représente 2 ou plus, les L₁ puissent être identiques ou différents l'un de l'autre ; et
chacun de R et R₁ à R₃ représente indépendamment l'hydrogène, un groupe alkyle ayant 1 à 20 atomes de carbone, un groupe aralkyle ayant 7 à 38 atomes de carbone, un groupe alcényle ayant 2 à 20 atomes de carbone, un groupe alcynyle ayant 2 à 20 atomes de carbone, un groupe dialkylamino ayant 2 à 40 atomes de carbone, un groupe diarylamino ayant 12 à 44 atomes de carbone, un groupe diaralkylamino ayant 14 à 76 atomes de carbone, un groupe acyle ayant 2 à 20 atomes de carbone, un groupe acyloxy ayant 2 à 20 atomes de carbone, un groupe alcoxy ayant 1 à 20 atomes de carbone, un groupe alcoxycarbonyle ayant 2 à 20 atomes de carbone, un groupe alcoxycarbonyloxy ayant 2 à 20 atomes de carbone, un groupe alkylsulfonyle ayant 1 à 20 atomes de carbone, un groupe hydrocarboné aromatique ayant 6 à 24 atomes de carbone, ou un groupe hétérocyclique aromatique ayant 3 à 16 atomes de carbone, et chacun peut porter un substituant ; 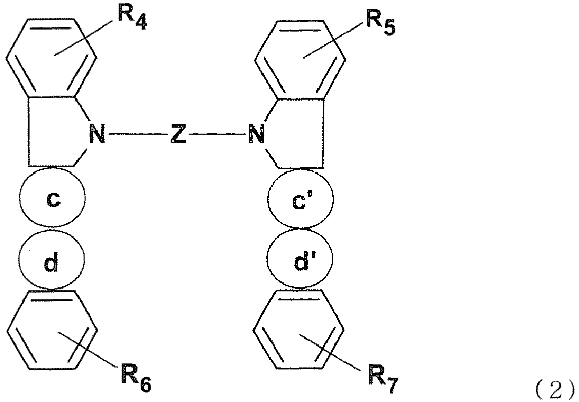 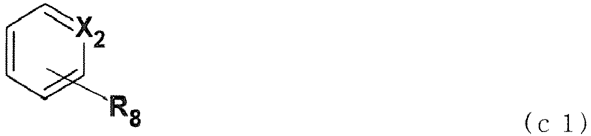 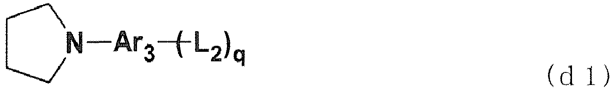
formules dans lesquelles :
chacun du cycle c et du cycle c' représente un cycle aromatique ou un hétérocycle représenté par la formule (c1) qui est fusionné au niveau de positions arbitraires de cycles adjacents, chacun du cycle d et du cycle d' représente un hétérocycle représenté par la formule (d1) qui est fusionné au niveau de positions arbitraires de cycles adjacents, et le cycle c et le cycle c' ou bien le cycle d et le cycle d' peuvent être identiques ou différents l'un de l'autre ;
X₂ représente C-R' ou N, Z représente un groupe hydrocarboné aromatique ayant 6 à 24 atomes de carbone, un groupe hétérocyclique aromatique ayant 3 à 16 atomes de carbone, ou un groupe aromatique lié divalent obtenu par liaison de 2 à 10 cycles aromatiques des groupes, Ar₃ représente un groupe hydrocarboné aromatique ayant 6 à 24 atomes de carbone, ou un groupe hétérocyclique aromatique monocyclique ayant 3 à 6 atomes de carbone, L₂ représente un groupe hydrocarboné aromatique ayant 6 à 24 atomes de carbone, un groupe hétérocyclique aromatique ayant 3 à 18 atomes de carbone, ou un groupe aromatique lié obtenu par liaison de 2 à 10 cycles aromatiques des groupes, et le groupe hydrocarboné aromatique, le groupe hétérocyclique aromatique, ou le groupe aromatique lié dans Z, Ar₃ et L₂ peut porter un substituant ;
q représente un entier de 0 à 7, sous réserve que, lorsque q représente 2 ou plus, les L₂ puissent être identiques ou différents l'un de l'autre ; et
chacun de R' et R₄ à R₈ représente indépendamment l'hydrogène, un groupe alkyle ayant 1 à 20 atomes de carbone, un groupe aralkyle ayant 7 à 38 atomes de carbone, un groupe alcényle ayant 2 à 20 atomes de carbone, un groupe alcynyle ayant 2 à 20 atomes de carbone, un groupe dialkylamino ayant 2 à 40 atomes de carbone, un groupe diarylamino ayant 12 à 44 atomes de carbone, un groupe diaralkylamino ayant 14 à 76 atomes de carbone, un groupe acyle ayant 2 à 20 atomes de carbone, un groupe acyloxy ayant 2 à 20 atomes de carbone, un groupe alcoxy ayant 1 à 20 atomes de carbone, un groupe alcoxycarbonyle ayant 2 à 20 atomes de carbone, un groupe alcoxycarbonyloxy ayant 2 à 20 atomes de carbone, un groupe alkylsulfonyle ayant 1 à 20 atomes de carbone, un groupe hydrocarboné aromatique ayant 6 à 24 atomes de carbone, ou un groupe hétérocyclique aromatique ayant 3 à 16 atomes de carbone, et chacun peut porter un substituant ; 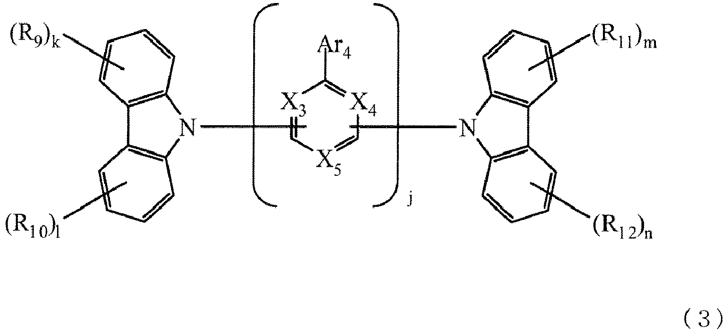
formule dans laquelle :
chacun de R₉ à R₁₂ représente indépendamment un groupe hydrocarboné aromatique ayant 6 à 18 atomes de carbone ou un groupe hétérocyclique aromatique ayant 3 à 9 atomes de carbone, et chacun peut porter un substituant ;
chaque Ar₄ représente indépendamment l'hydrogène ou un groupe hydrocarboné aromatique ayant 6 à 24 atomes de carbone, et le groupe hydrocarboné aromatique peut porter un substituant, j représente un entier de 1 ou 3, et au moins un Ar₄ ne représente pas l'hydrogène ;
chacun de X₃ à X₅ représente indépendamment N, C-R", ou C-, et chaque R" représente indépendamment l'hydrogène, un groupe alkyle ayant 1 à 20 atomes de carbone, un groupe alcoxy ayant 1 à 20 atomes de carbone, ou un groupe diarylamino ayant 12 à 44 atomes de carbone ; et
k, l, m et n représentent des entiers satisfaisant à 0 ≤ k+l+m+n ≤ 16.

2. Dispositif électroluminescent organique selon la revendication 1, dans lequel un des deux matériaux hôtes comprend un matériau hôte choisi parmi les composés représentés chacun par l'une quelconque des formules générales (1) et (2), un autre des deux matériaux hôtes comprend un matériau hôte choisi parmi les composés représentés chacun par la formule générale (3), et la différence d'affinité électronique (ΔEA) entre les deux matériaux hôtes est supérieure à 0,1 eV.

3. Dispositif électroluminescent organique selon la revendication 1, dans lequel, dans la formule générale (1), X₁ représente C-R.

4. Dispositif électroluminescent organique selon la revendication 1, dans lequel, dans la formule générale (2), X₂ représente C-R'.

5. Dispositif électroluminescent organique selon la revendication 1, dans lequel, dans la formule générale (1), au moins l'un de Ar₁ et Ar₂ représente un groupe hétérocyclique aromatique substitué ou non substitué ayant 3 à 9 atomes de carbone.

6. Dispositif électroluminescent organique selon la revendication 1, dans lequel, dans la formule générale (3), j représente l'entier 1.

7. Dispositif électroluminescent organique selon la revendication 1, dans lequel, dans la formule générale (3), chacun de X₃ à X₅ représente C-H, N, ou C-.

8. Dispositif électroluminescent organique selon la revendication 1, dans lequel la différence de température d'évaporation entre le matériau hôte (H1) et le matériau hôte (H2) est de l'ordre de 30°C.

9. Dispositif électroluminescent organique selon la revendication 8, dans lequel la différence de température d'évaporation est de l'ordre de 10°C.

10. Dispositif électroluminescent organique selon l'une quelconque des revendications 1 à 9, dans lequel le dopant luminescent comprend un dopant luminescent phosphorescent comprenant un complexe organométallique contenant au moins un métal choisi parmi le ruthénium, le rhodium, le palladium, l'argent, le rhénium, l'osmium, l'iridium, le platine, et l'or.

11. Méthode pour préparer un dispositif électroluminescent organique selon la revendication 1, dans laquelle le dispositif électroluminescent organique comprend une couche luminescente [5], dans laquelle la couche luminescente [5] est produite par mélange au préalable du matériau hôte (H1) et du matériau hôte (H2), et dépôt en phase vapeur du mélange à partir d'une source de dépôt en phase vapeur.
